# EUROPEAN PATENT APPLICATION

(11) **EP 3 988 088 A1**
(43) Date of publication of application: **27.04.2022**
(21) Application number: 21202865.8
(22) Date of filing: 22.10.2013
(51) Int. Cl.: A61K 9/08, A61K 9/06, A61K 47/10, A61K 47/20, A61K 47/38, A61K 31/196, A61P 19/02, A61J 1/00, A61M 35/00, B05B 11/04, B65D 83/76, G01F 11/02, F04B 9/14

(54) **DISPENSING SYSTEM**

(30) Priority: 22.10.2012 US 201213657711
(62) Divisional of application: 13849262.4
(71) Applicant: Nuvo Pharmaceuticals Inc., Mississauga, ON L5N 6J5 (CA)
(72) Inventor: Desai, Tejas, Mississauga, L5M 0K7 (CA); Vo, Ngoc Truc-Chi, Longueuil, J4J 5H1 (CA); Doty, Brian Dean, Saint Charles, 63303 (US); Brendel, Ronald, J., Florissant, 63031 (US); Yue, Baohua, Ballwin, 63021 (US)
(74) Representative: Moré, Solveig Helga

(57) **Abstract**

The invention provides systems and methods for controlled dispensing of topical analgesics contained in a metered dispensing system. The systems and method are useful for treating signs and symptoms of osteoarthritis. The method includes depressing a hand pump to dispense a dose of a topical pain reliever in a viscous solution from the hand pump, wherein the hand pump is configured to dispense the dose within a tolerance specified by a corresponding label approved by a government regulatory agency; and spreading the topical solution on skin.

## Description

### CROSS-REFERENCES TO RELATED APPLICATIONS

This application is a continuation of and claims the benefit of U.S. Patent Application No. 13/657,711, filed October 22, 2012, which is hereby incorporated by reference in its entirety for all purposes.

### FIELD OF THE INVENTION

This invention relates to systems and methods for delivering topical medications in a metered dose.

### BACKGROUND OF THE INVENTION

Topical analgesics are available in many dosage forms, including solutions, liquids, foams, gels, creams, ointments and the like. They are packaged in various container closure systems, such as tubes, bottles, pouches, and canisters. One common issue with many topical analgesic products is inaccurate unit dose dispensing from multi-dose containers, which may cause patient underdoses or overdoses.

A dosing card is a known method to measure topical semi-solid drugs. The commercially available Voltaren^{®} antiphlogistics and antirheumatics in the form of gels is an example that uses a dosing card as the primary dose measurement method. When the medicine gel is squeezed out of a tube to cover a pre-specified area on the dosing card, it is assumed that the correct dose is dispensed. Although the specified area serves as a guide, it is not always easy for the users to cover the exact area every time, and its two dimensional nature also limits the dispensing accuracy.

What is needed in the art are new ways to deliver viscous transdermal medicines administered topically. Methods are needed that will allow accurate and convenient dosing of a viscous solution or gel for local topical analgesics. Methods for improving dosing accuracy are desired to prevent wrong dose quantities. The present invention satisfies these and other needs.

### BRIEF SUMMARY OF THE INVENTION

This present invention provides systems and methods for administering topical agents. As such, in one embodiment, the present invention provides: a method for administering a recommended dosage of a topical pain reliever in a viscous solution, the method comprising:
actuating a hand pump, the actuating:
a) causing a tappet having a rod connected thereto to compress a spring and the rod to slide with respect to a piston and enter a metering chamber holding a first predetermined amount of topical pain reliever in the viscous solution;
b) causing an outlet valve of the metering chamber to open, which allows the first predetermined amount of topical pain reliever in the viscous solution to flow into a channel or opening of the rod; and then
c) causing the piston to stroke when a limit stop of the tappet connects with a limit stop of the piston, wherein the first predetermined amount of topical pain reliever in the viscous solution is evacuated through an output duct of the tappet, which rises back up by action of the spring; and then
d) causing the outlet value of the metering chamber to close and create a vacuum inside the metering chamber and to fill the chamber;
e) optionally repeating steps a) through d) to deliver the recommended dose; and
f) spreading the viscous solution on skin.

In another embodiment, the present invention provides a method for treating the signs and symptoms of osteoarthritis, the method comprising:
depressing a hand pump to dispense 1/n of a dose of a topical pain reliever in a viscous solution from the hand pump, wherein n is a positive integer, wherein the hand pump is configured to dispense the 1/n of the dose within a tolerance specified by a corresponding label approved by a government regulatory agency; and spreading the topical solution on skin.

In yet another embodiment, the present invention provides a dispensing system for a topical pain reliever in a viscous solution, the system comprising:
a topical pain reliever in a viscous solution comprising sodium diclofenac;
a sealed bag holding the viscous solution; and
a hand pump in fluid communication with the viscous solution, the hand pump comprising:
   a pump housing defining a metering chamber;
   a tappet having a rod connected thereto to compress a spring and slide along a piston and enter a metering chamber holding a first predetermined amount of topical pain reliever in a viscous solution, wherein the metering chamber is sized for a throughput of 1/n dose of the sodium diclofenac, wherein n is a positive integer, wherein the first predetermined amount of topical pain reliever in the viscous solution is evacuated through an output duct of the tappet, which rises back up by action of the spring to dispense the 1/n of the dose within a tolerance specified by a corresponding label approved by a government regulatory agency.

In still yet another embodiment, the present invention provides a method for treating the signs and symptoms of osteoarthritis of the knee of a human patient with a topical diclofenac preparation, the method comprising:
dispensing a therapeutically effective amount of diclofenac from a topical diclofenac preparation packaged in a pharmaceutically acceptable hand pump;
applying the therapeutically effective amount of diclofenac to the knee,
wherein the patient attains therapeutic blood levels of diclofenac within 4 to 12 hours after administration of the preparation and maintains pain relief for about 12 hours after administration of the preparation.

Advantageously, the present invention improves patient compliance. In certain aspects, the methods and systems herein include patient instructions to promote proper use of the container and accurate dosing. These and other aspects, objects, and advantages will become more apparent when read with the detailed description and drawings which follow.

### BRIEF DESCRIPTION OF THE DRAWINGS

**FIG.** 1 illustrates a hand pump suitable for use in the present invention.
**FIGS. 2A-2Z** illustrate a sample label that can be approved by a government regulatory agency. Each figure is one page of the same label.
**FIGS. 3A-3B** illustrate a run chart and a statistical graph summary, respectively, for the weight of a first conforming dose from each container (the next dose after the first product is discharged during priming).
**FIGS. 4A-4B** show capability analyses for unit dose weights at specification ranges of 0.8-1.2 g and 0.9-1.1 g, respectively.
**FIG.** 5 shows a statistical process control chart for unit dose weight.

### DETAILED DESCRIPTION

### I. Embodiments

The present invention provides methods of and systems for administering topical agents. In certain aspects, the methods and systems employ a non-pressurized system. In one embodiment, the present invention provides a method for administering a recommended dosage of a topical pain reliever in a viscous solution, the method comprising:
actuating a hand pump, the actuating:
a) causing a tappet having a rod connected thereto to compress a spring and the rod to slide with respect to a piston and enter a metering chamber holding a first predetermined amount of topical pain reliever in the viscous solution;
b) causing an outlet valve of the metering chamber to open, which allows the first predetermined amount of topical pain reliever in the viscous solution to flow into a channel or opening of the rod; and then
c) causing the piston to stroke when a limit stop of the tappet connects with a limit stop of the piston, wherein the first predetermined amount of topical pain reliever in the viscous solution is evacuated through an output duct of the tappet, which rises back up by action of the spring; and then
d) causing the outlet value of the metering chamber to close and create a vacuum inside the metering chamber and to fill the chamber;
e) optionally repeating steps a) through d) to deliver the recommended dose; and
f) spreading the viscous solution on skin.

In certain aspects, the topical pain reliever comprises sodium diclofenac. In certain aspects, the viscous solution or gel is about 1.5% to about 3% by weight of sodium diclofenac such as about 2% by weight of sodium diclofenac.

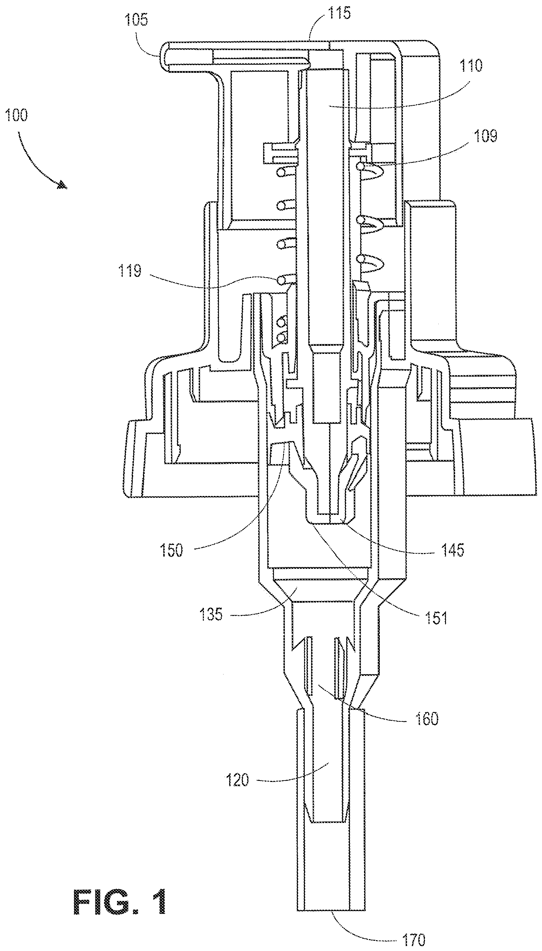

FIG. 1 is an illustration of a hand pump 100 suitable for use in the present invention. The system is available from Rexam PLC (of England and Wales) as a pump having SP943 as an identifier. A tappet 109 is activated by pressing the top 115 and compressing a spring 119. The tappet 109 is connected to a rod 110 having a channel 145, which slides along a piston 150 and enters a metering chamber 135. The metering chamber 135 is filled with a viscous solution, gel, or formulation. In operation, an outlet valve of the metering chamber opens, which allows the viscous solution, gel or formulation to flow into the channel or opening 145 from inlet 120. When a limit stop comes into contact with the piston 150, a venting hole allows the pressure inside the container to be the same as the atmospheric pressure. Then, with the piston 150 continuing its stroke, the viscous solution, gel or formulaion is evacuated through the tappet's output duct. Once the product has been evacuated, the tappet is released and rises back up by action of the spring. The outlet valve closes in this movement, since the rod returns to a water-tight position. A vacuum is created inside the metering chamber, which makes a ball check valve 160 rise and open the way for the viscous solution, gel, or formulation to be sucked up into the dosage chamber. At the end of the piston rising stroke, the venting hole and the piston chamber are no longer in communication, which restores the overall water-tightness of the system.

The metering chamber 135 communicates with a dip tube 170 by means of a ball check valve 160. The piston 150 slides inside the pump body and, more particularly, inside the metering chamber 135. In one embodiment, a pump as disclosed in U.S. Patent No. 7,243,820, issued July 17, 2007, hereby incorporated by reference, is suitable for use in the present invention.

In certain aspects, the method further comprises aspirating a second predetermined amount of a topical pain reliever from a sealed bag holding the viscous solution, thereby shrinking the bag. In certain instances, pump 100 is disposed above the bag with dip tube 170 inserted in the bag. Preferably, the bag is metal lined or coated with polyester or polyethylene. Preferably, the metal pouch is inert to the pain reliever formulation. The metal can be made of aluminum or an aluminum alloy. In certain instances, the hand pump is elongated and the actuating occurs when the elongated hand pump is held at an angle with respect to a gravitational vector. In other instances, the hand pump is elongated and the actuating occurs when the elongated hand pump is upside-down with respect to a gravitational vector. An example of the liner is in the Rexam Sof'Bag, which is a foil laminate bag as an inner collapsible package.

In another embodiment, the present invention provides a method for treating signs and symptoms of osteoarthritis, the method comprising:
depressing a hand pump to dispense 1/n of a dose of a topical pain reliever in a viscous solution from the hand pump, wherein n is a positive integer, wherein the hand pump is configured to dispense the 1/n of the dose within a tolerance specified by a corresponding label approved by a government regulatory agency (*e.g*. United States Food and Drug Administration (FDA); and then
spreading the topical solution on skin.

In certain instances, the dose tolerance is about ±0-20 %, such as ± 0, 1, 2, 3, 4, 5, 6, 7, 8, 9, 10, 11,12, 13, 14, 15, 16, 17, 18, 19, or 20%. In other instances, the dose tolerance is about ±5-10%. The term "dose tolerance" includes the accuracy or precision of the first dose compared to subsequent dose, or as otherwise known in the art. For example, if the first dose is 1 g, a ±10% dose tolerance is between 0.9 to 1.1 g.

In certain instances, the integer "n" has a value from about 1 to about 10 such as 1, 2, 3, 4, 5, 6, 7, 8, 9 or 10. In one instance, n = 1 and the depressing the hand pump a single time dispenses an entire dose of the viscous topical pain reliever in a viscous solution. In other instances, n = 2, wherein the depressing of the hand pump twice dispenses a half-daily dose of the viscous topical pain reliever in a viscous solution. A daily dose may be two applications of 2 mL, or 4 actuations total.

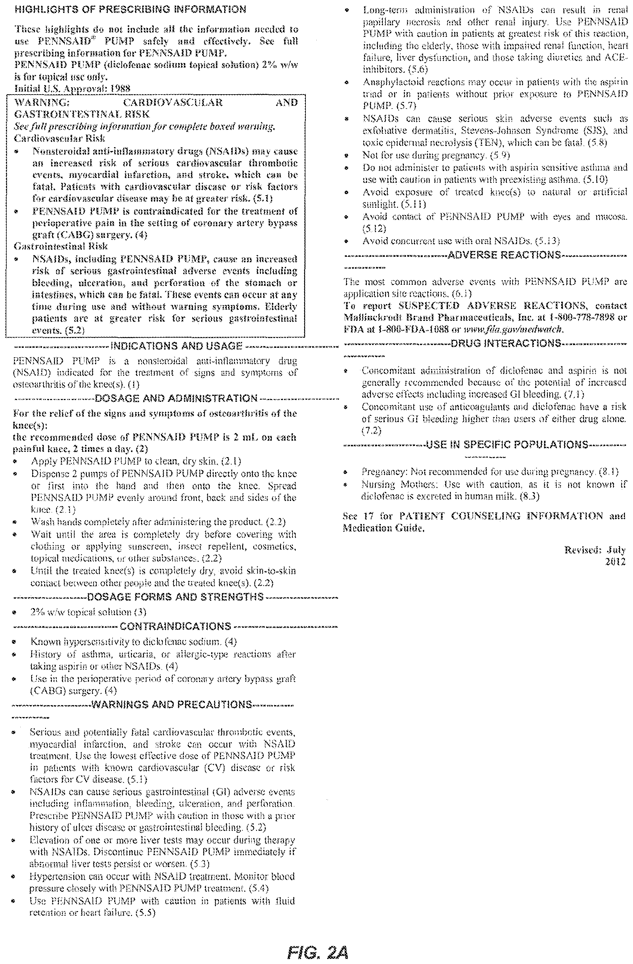

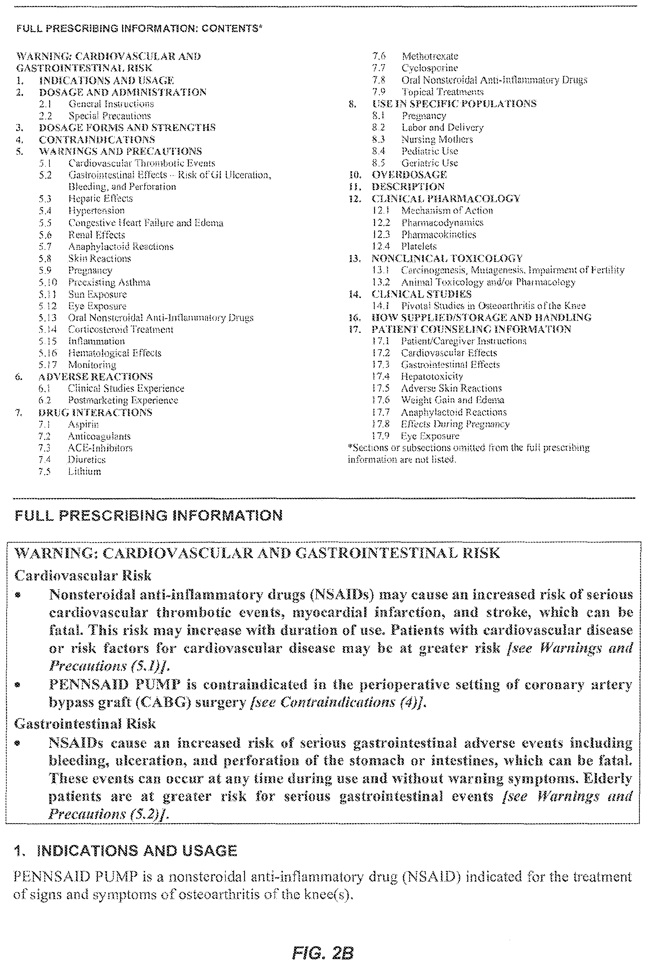

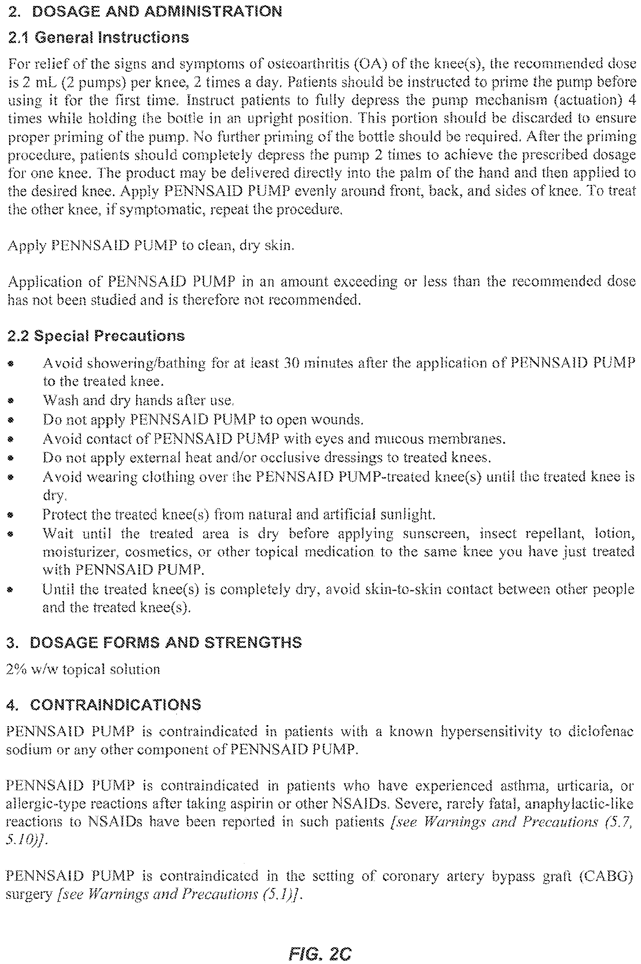

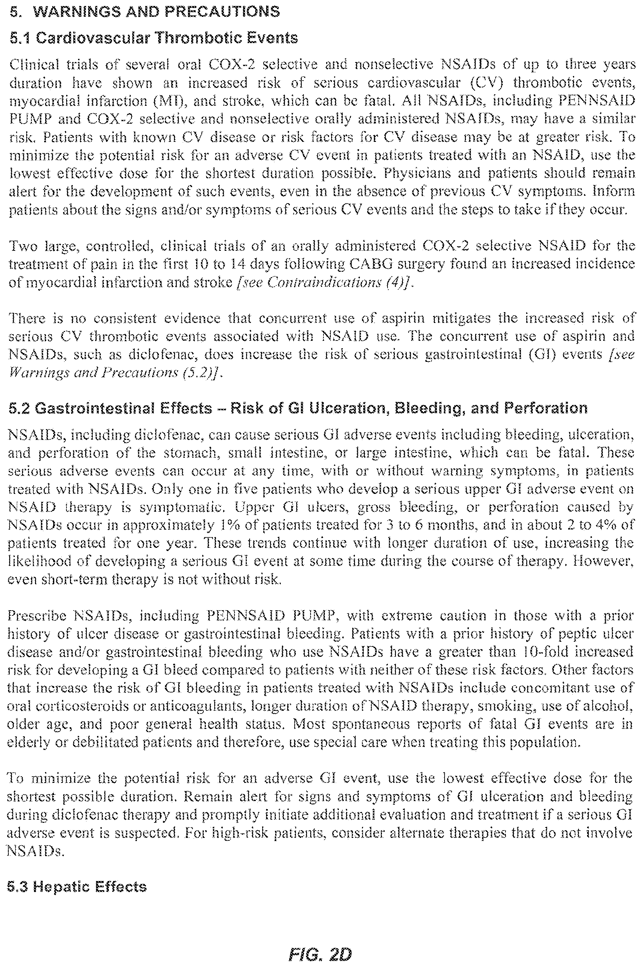

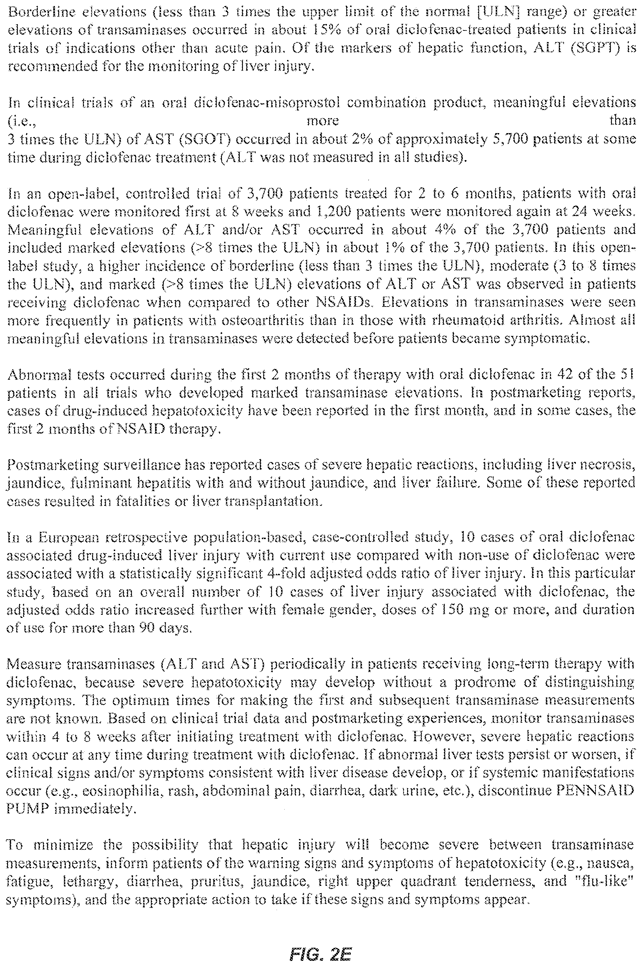

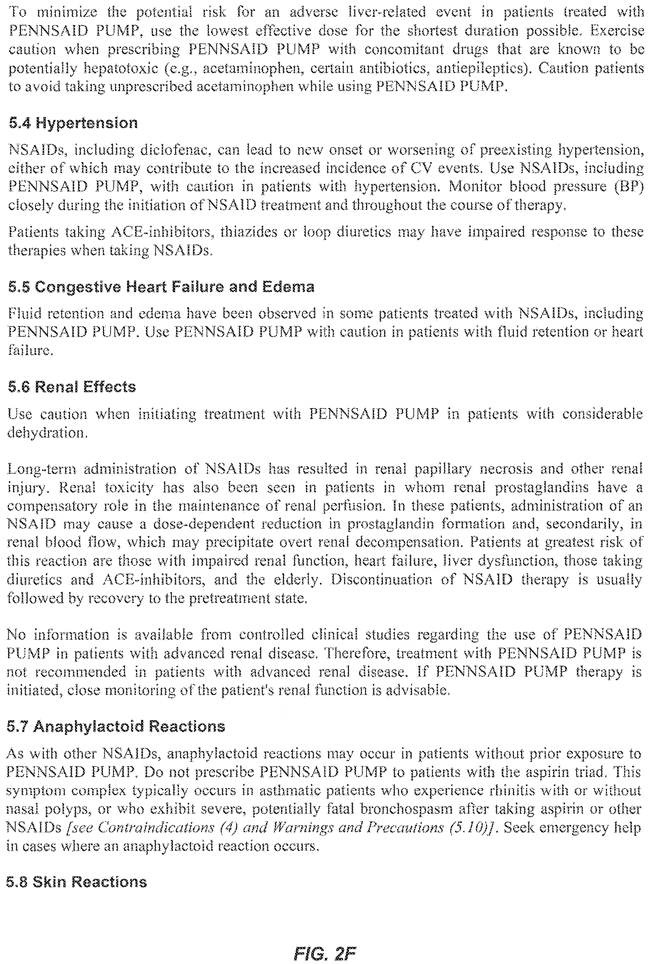

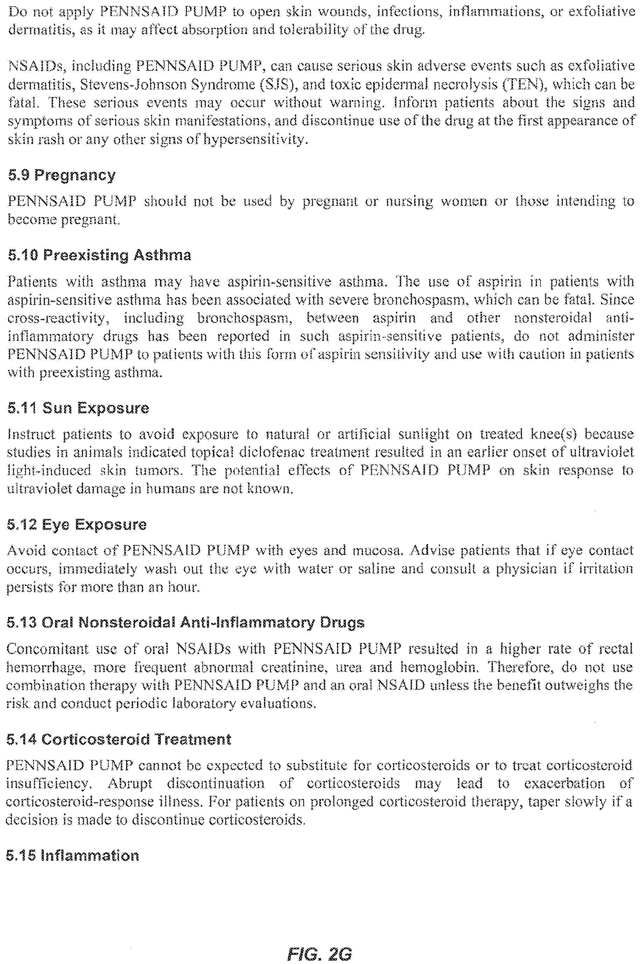

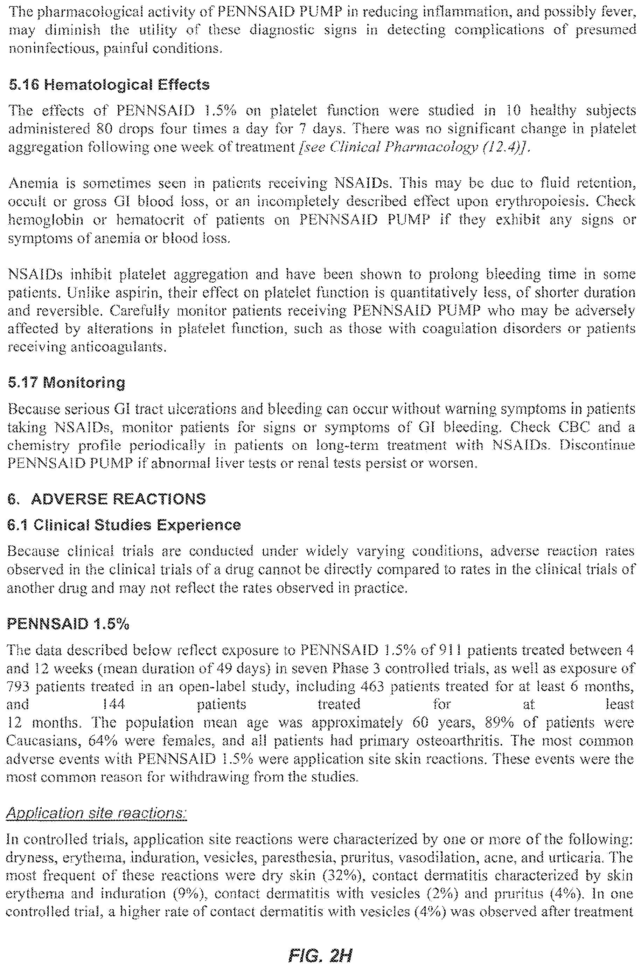

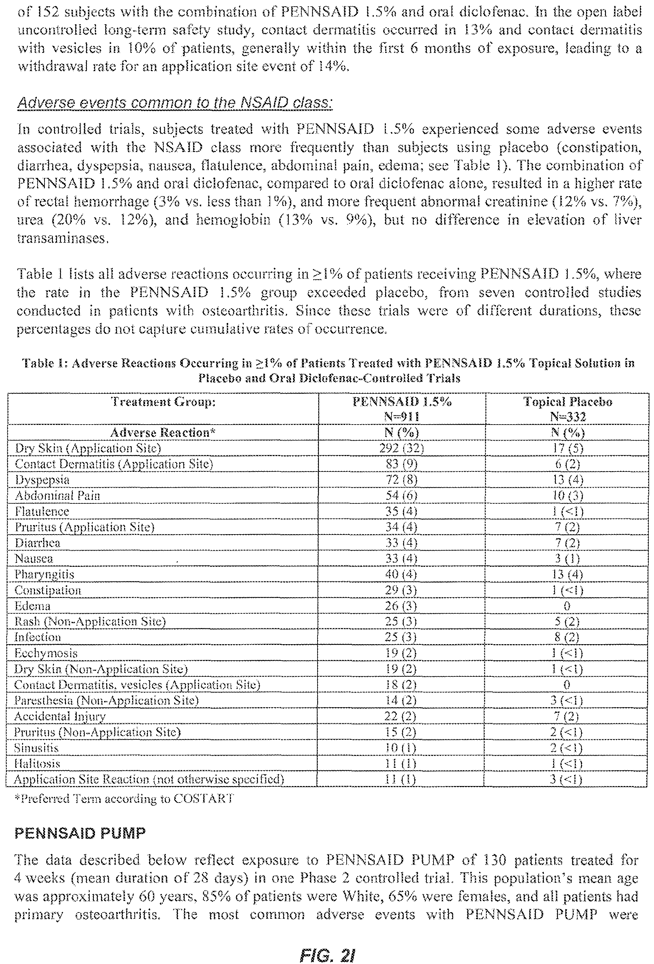

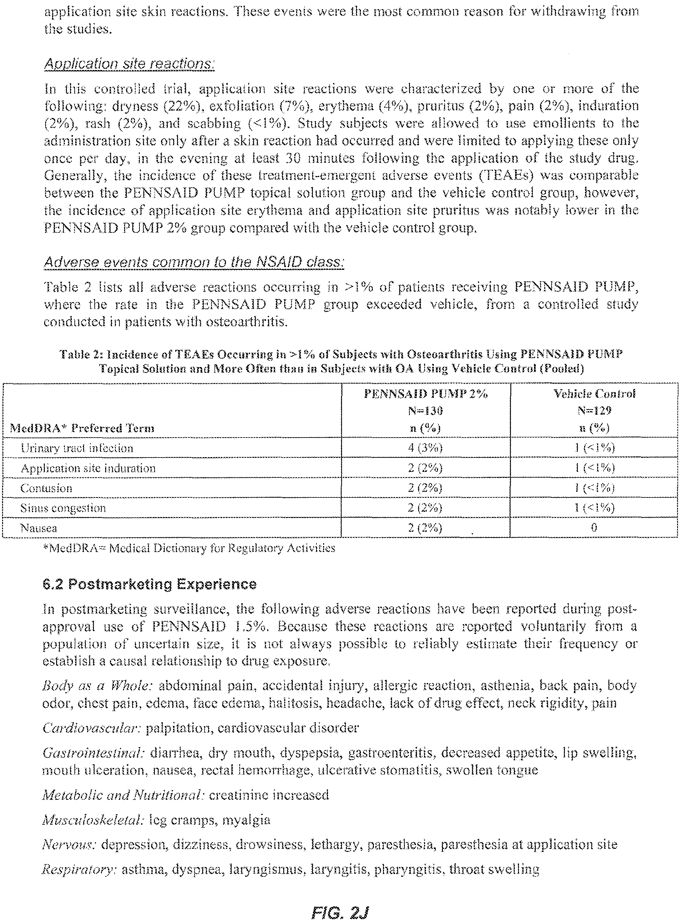

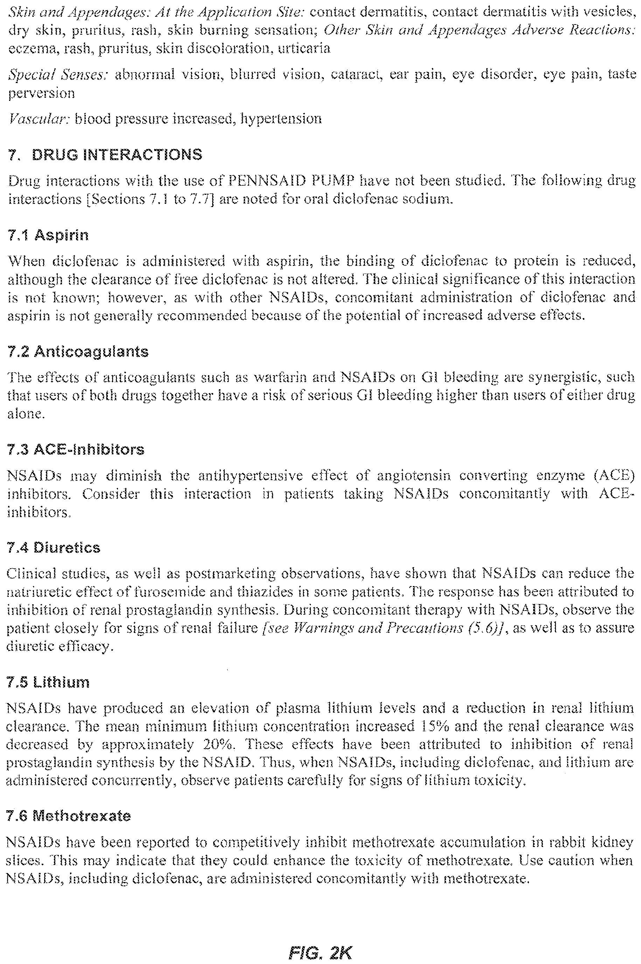

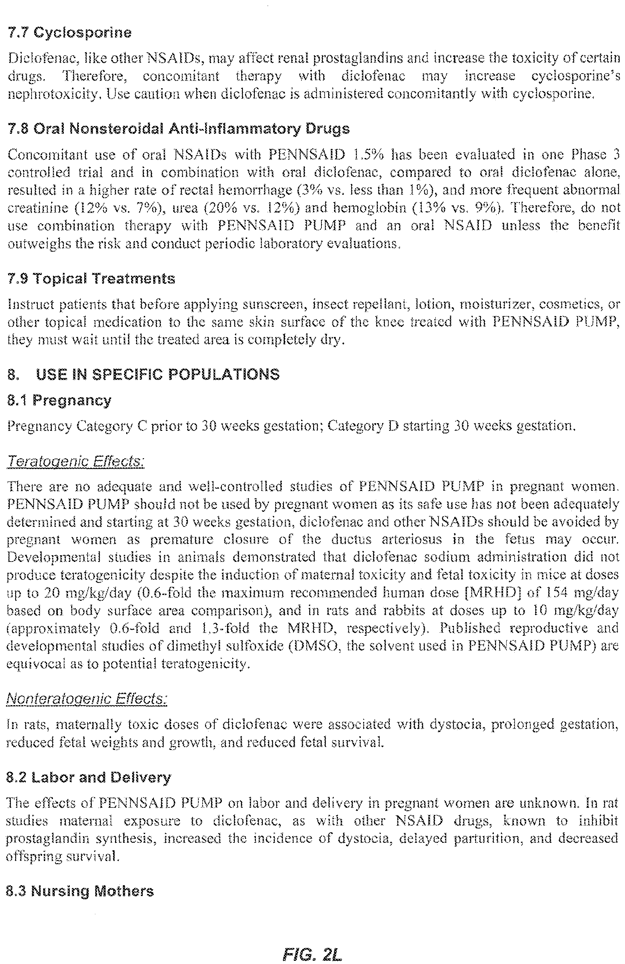

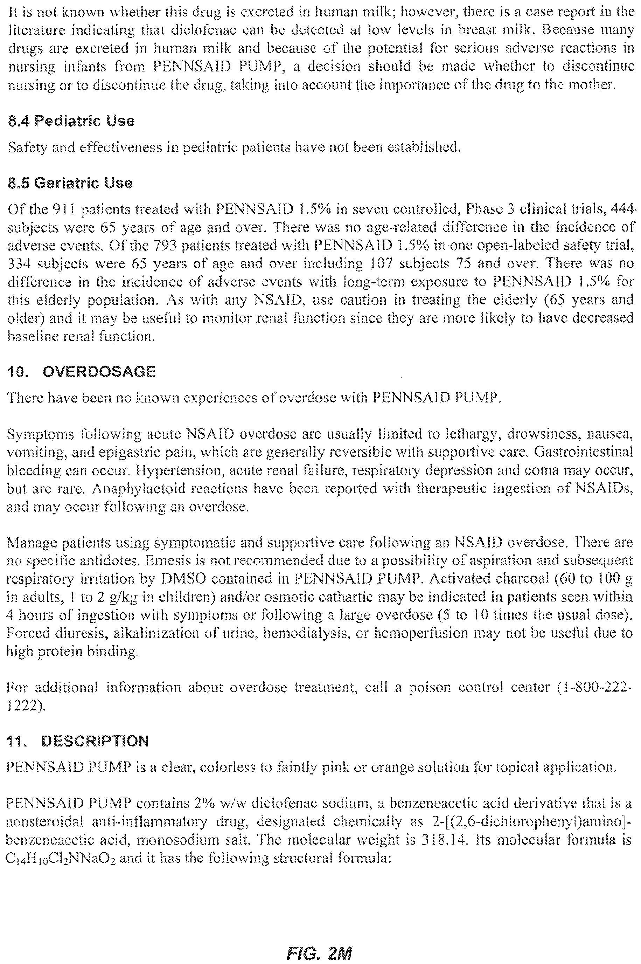

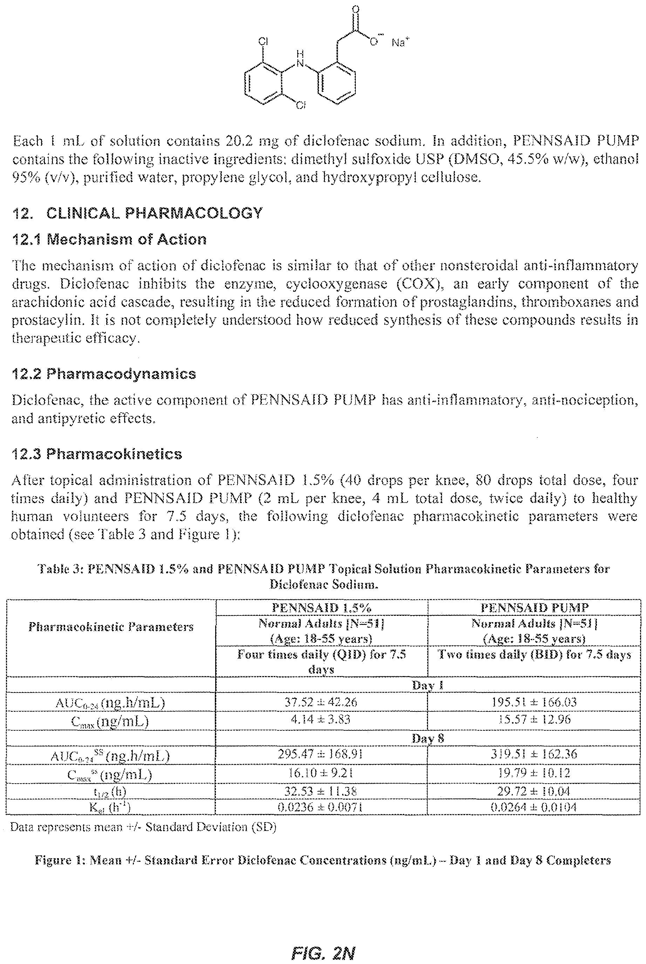

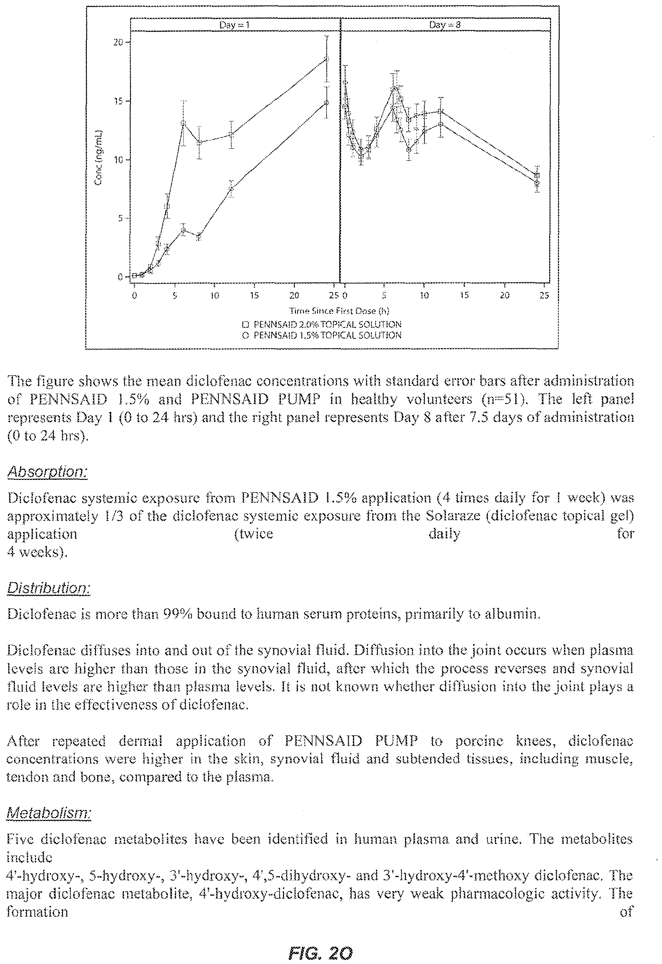

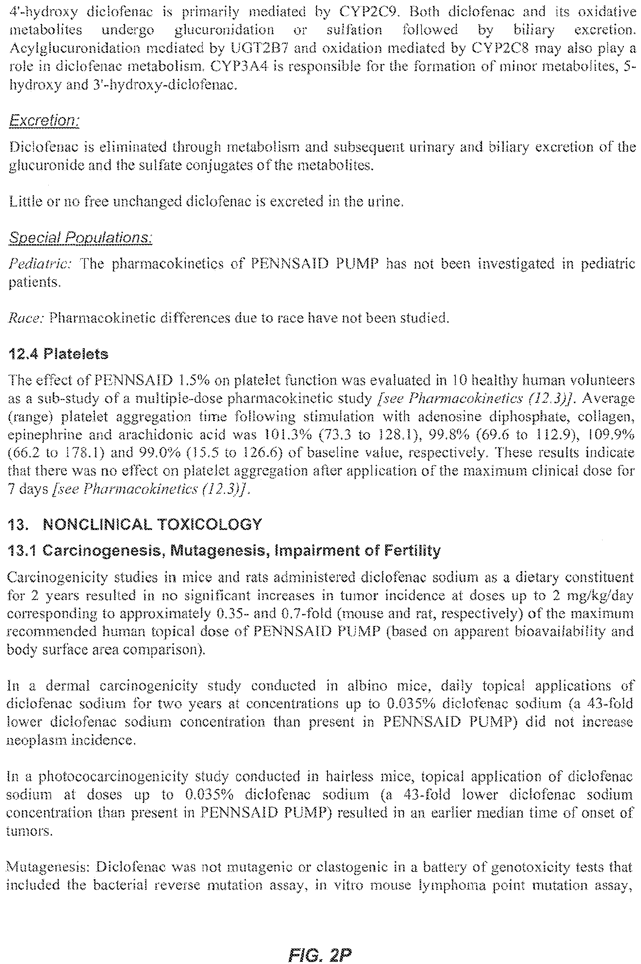

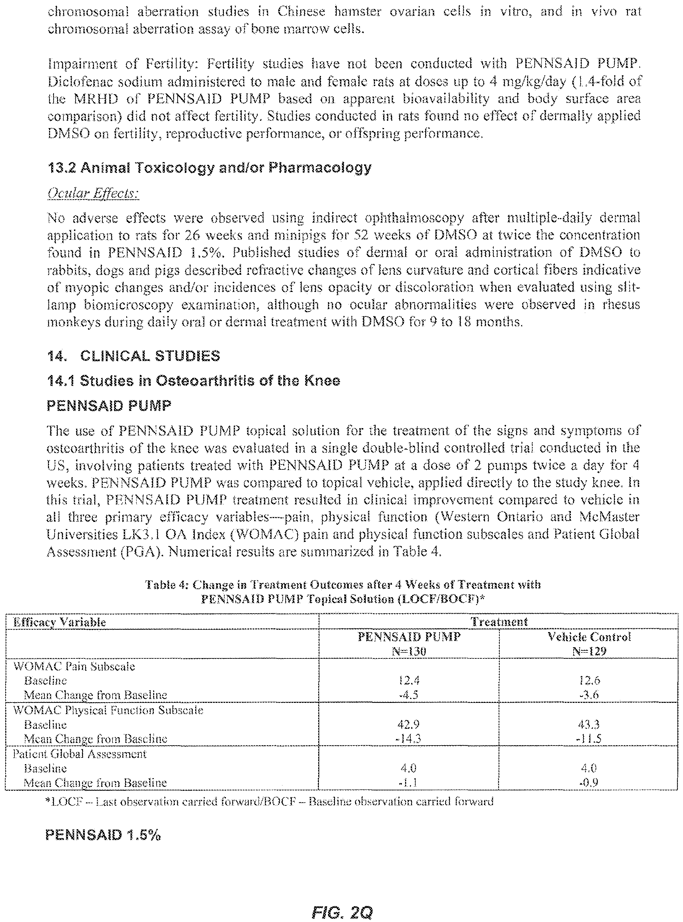

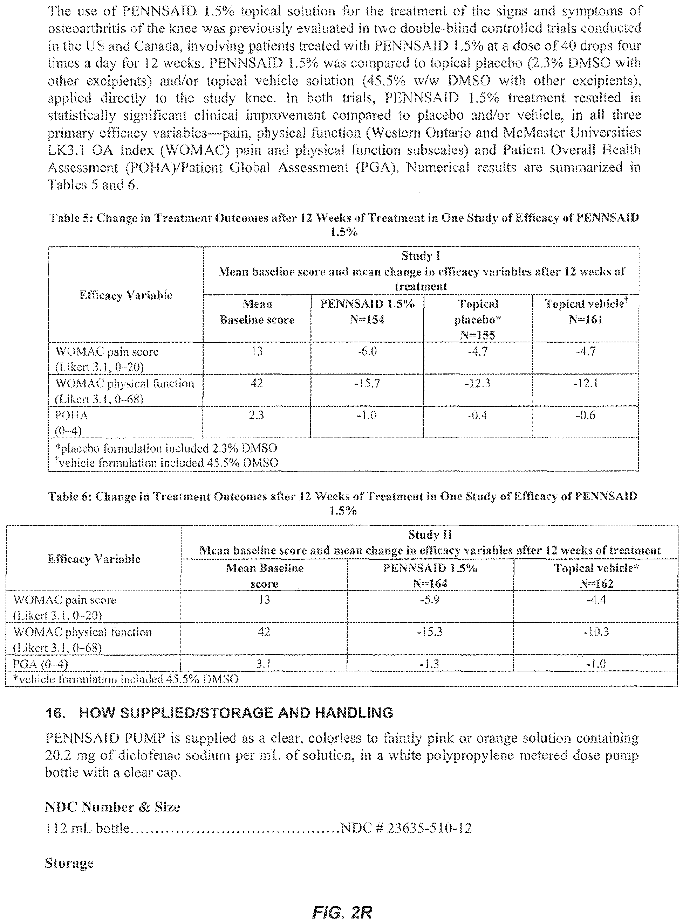

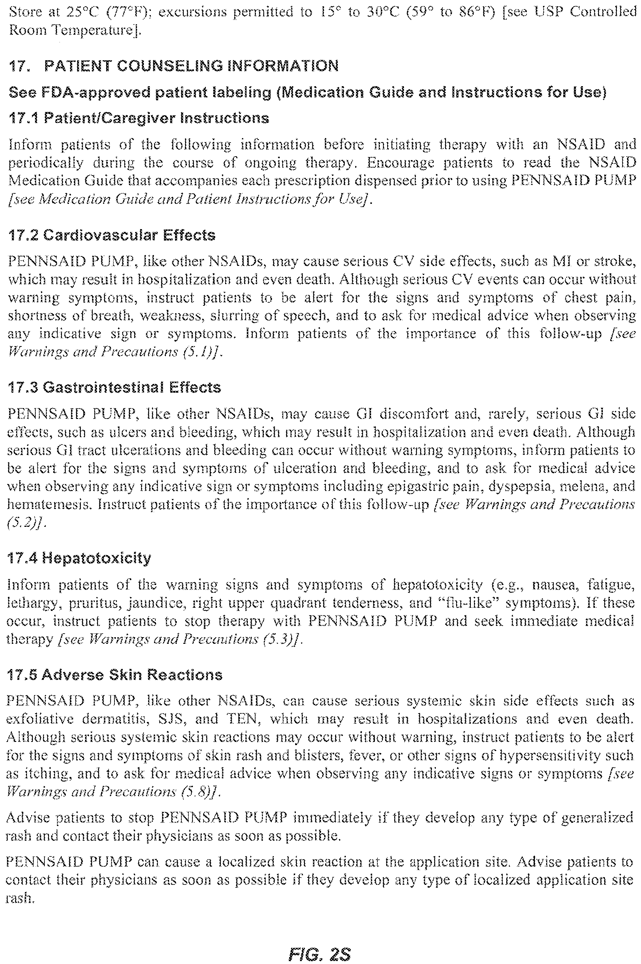

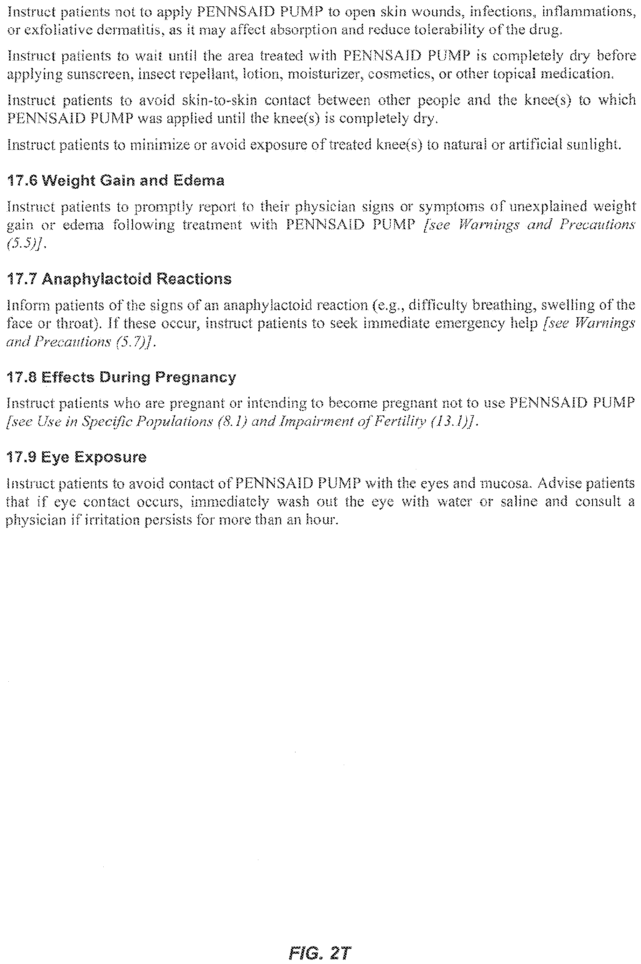

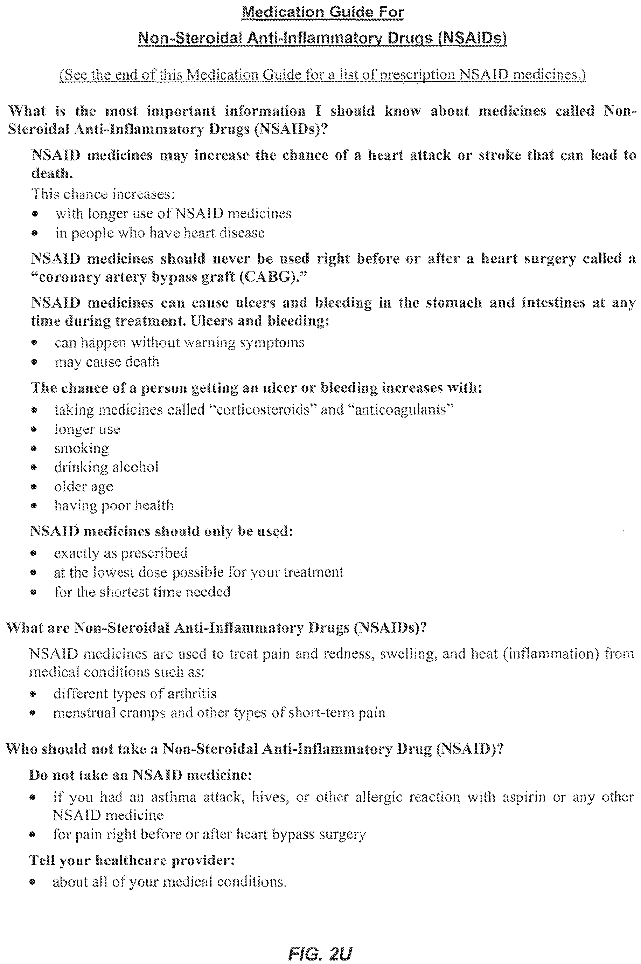

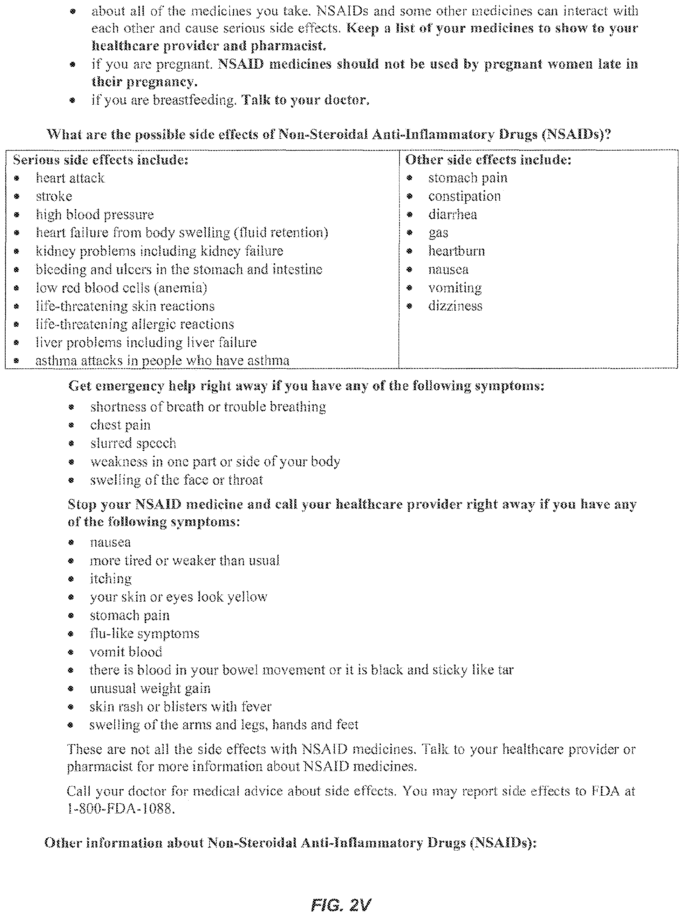

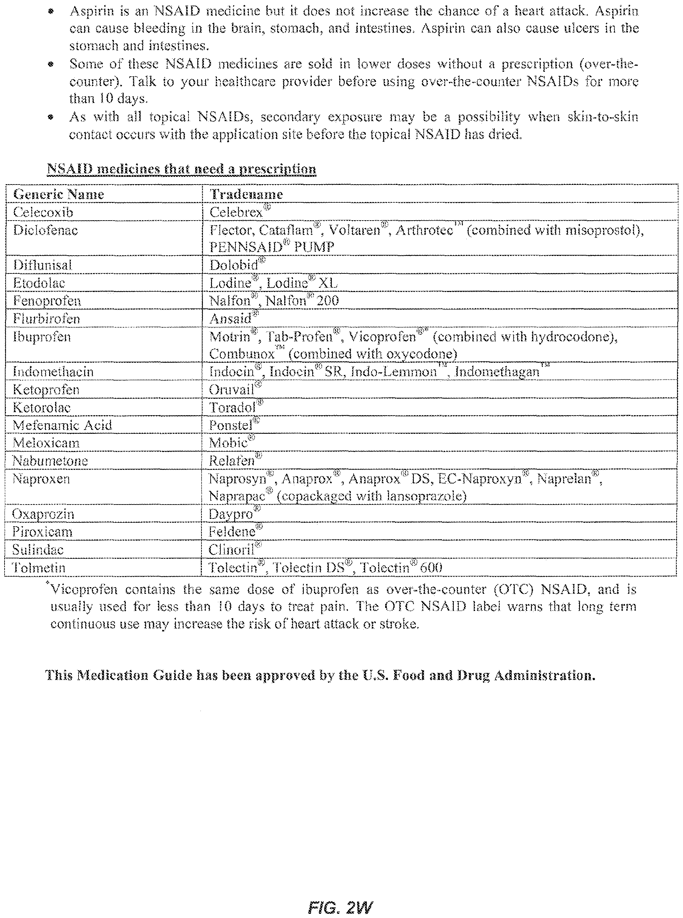

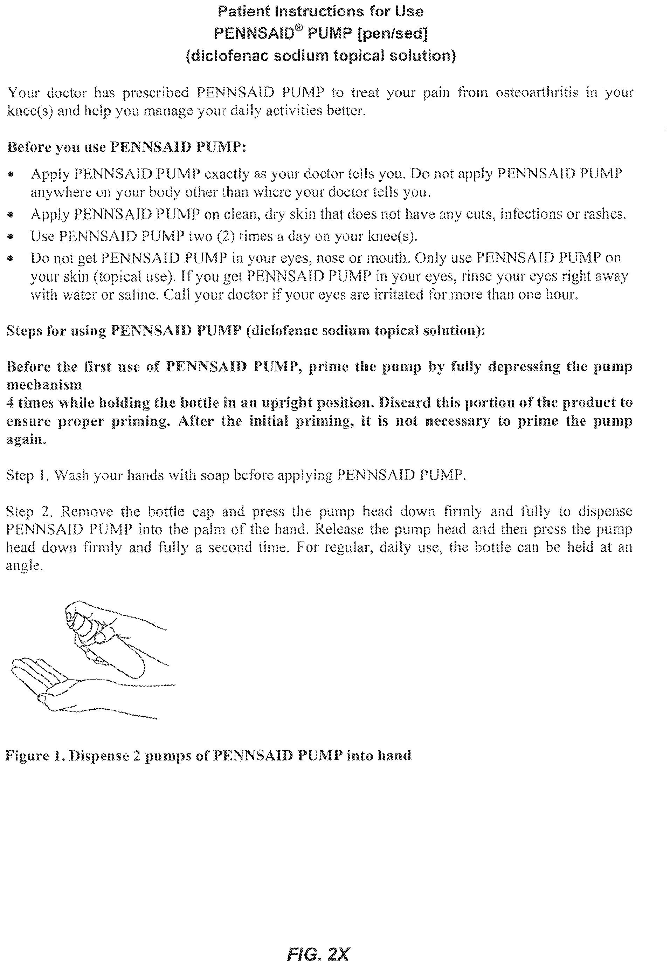

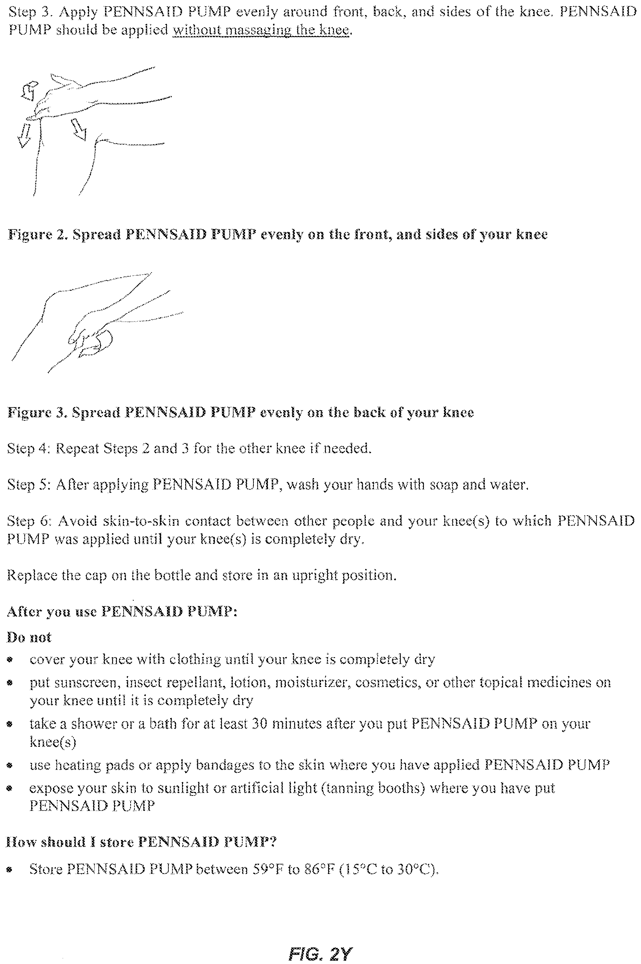

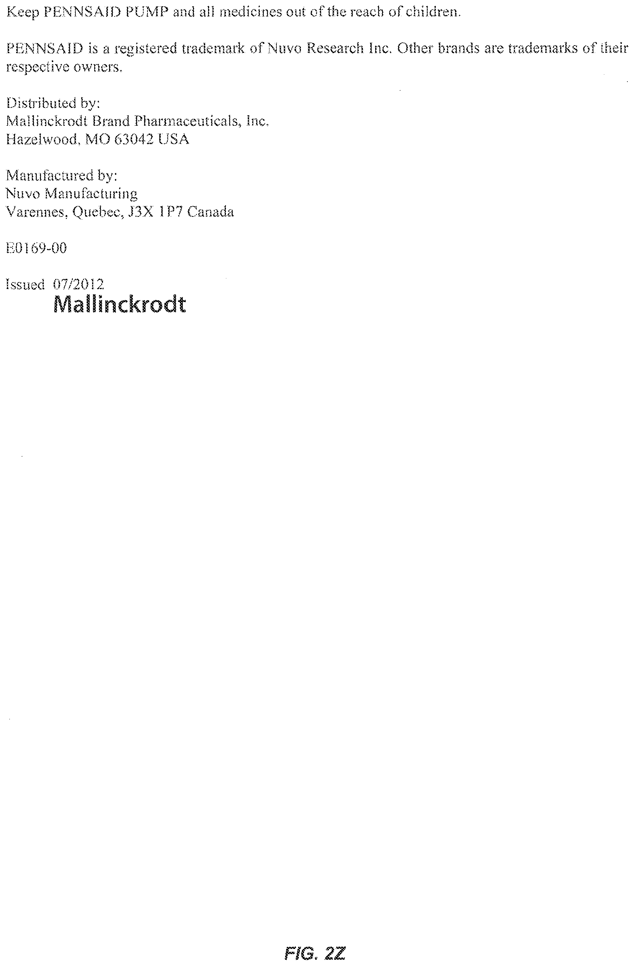

FIGS. 2A-2Z illustrate a sample label that can be approved by a government regulatory agency. Such labels can include instructions for a pain reliever that are provided or distributed with the pain reliever, to patients, doctors, pharmacists, etc. The sample label in the figure is for diclofenac sodium topical solution in a pump in accordance with an embodiment of the invention.

In yet another embodiment, the present invention provides a dispensing system for a topical pain reliever in a viscous solution, the system comprising:
a topical pain reliever in a viscous solution comprising sodium diclofenac;
a sealed bag holding the viscous solution; and
a hand pump in fluid communication with the viscous solution, the hand pump comprising:
   a pump housing defining a metering chamber;
   a tappet having a rod connected thereto to compress a spring and slide along a piston and enter a metering chamber holding a first predetermined amount of topical pain reliever in a viscous solution, wherein the metering chamber is sized for a throughput of 1/n dose of the sodium diclofenac, wherein n is a positive integer, wherein the first predetermined amount of topical pain reliever in a viscous solution is evacuated through an output duct of the tappet, which rises back up by action of the spring to dispense the 1/n of the dose within a tolerance specified by a corresponding label approved by a government regulatory agency.

In certain preferred aspects, the sealed bag holding the viscous solution excludes air, thereby allowing the hand pump and sealed bag to dispense the viscous solution from any orientation with respect to a gravity vector. Preferably, the system further comprises a bottle, a can or other canister enclosing the bag.

A "gravity vector" includes a direction in which gravity exerts a force on matter or as otherwise known in the art.

### II. Topical Formulation

In certain aspects, the formulation or viscous solution of topical pain reliever is a non-steroidal anti-inflammatory ("NSAID") drug or pharmaceutically acceptable salt thereof. More preferably, the non-steroidal anti-inflammatory is diclofenac, which can exist in a variety of salt forms, including sodium, potassium, or diethylamine forms. In a preferred embodiment, the sodium salt of diclofenac is used. Diclofenac sodium or sodium diclofenac can be present in a range of approximately about 0.1% to about 10%, such as about 1, 2, 3, 4, or 5% w/w. In a preferred aspect, the pump solution contains a viscous solution, or gel of is 2% w/w (mass/mass) diclofenac sodium.

"About" includes within a tolerance level of ±1%, ±2%, ±3%, ±4%, ±5%, ±10%, ±15%, ±20%, ±25%, or as otherwise known in the art.

In certain aspects, the present active ingredient formulation or viscous solution includes a penetration enhancer. The penetration enhancer can be dimethyl sulfoxide ("DMSO") or derivatives thereof. The DMSO may be present in an amount by weight of about 1% to about 70%, and more preferably, between about 25% and about 60%, such as about 25, 30, 40, 45, 50, 55, or 60% w/w. Preferably, DMSO is used in the present invention at a concentration of about 40 to about 50% w/w, such as about 41, 42, 43, 44, 45, 46, 47, 48, 49 and 50% and all fractions in between such as about 44, 44.5, 45, 45.5, 46, 46.5%, and the like.

In certain aspects, the present invention includes a lower alkanol, such as methanol, ethanol, propanol, butanol or mixtures thereof. In certain embodiments, the alkanol is present at about 1 to about 50% w/w. Preferably, ethanol is used at about 1-50% w/w, such as 1, 5, 10, 15, 20, 25, 30, 35, 40, 45, or 50% w/w, and all fractions in between.

In certain aspects, the present invention includes a polyhydric alcohol, such as a glycol. Suitable glycols include ethylene glycol, propylene glycol, butylene glycol, dipropylene glycol, hexanetriol and a combination thereof. Preferably, propylene glycol is used at about 1-15% w/w, such as about 1, 2, 3, 4, 5, 6, 7, 8, 9, 10, 11, 12, 13, 14, or 15% w/w, and all fractions in between.

In certain embodiments, the present invention includes glycerol (also referred to herein as glycerin) at a concentration of 0-12% w/w. Preferably, glycerol is used at 1-4% w/w, such as about 1, 2, 3, or 4 % w/w, and all fractions in between. In some embodiments, no glycerol is used in the formulation *i.e.,* the viscous solution or gel optionally comprises glycerol.

In a preferred aspect, the topical pain reliever comprising a diclofenac solution has at least one thickening agent to make a viscous solution. The at least one thickening agent of the present invention may be an acrylic polymer (for example, Carbopol polymers, Noveon polycarbophils and Pemulen polymeric emulsifiers available commercially from Noveon Inc. of Cleveland, Ohio), an acrylic polymer derivative, a cellulose polymer, a cellulose polymer derivative, polyvinyl alcohol, poloxamers, polysaccharides or mixtures thereof. Preferably the at least one thickening agent is hydroxypropylcellulose (HPC) used such that the end viscosity is between about 10 and about 50000 centipoise (cps). More preferably the end viscosity is between about 500 and about 20000 cps or about 500-3000 cps or about 1000-2000 cps. In certain aspects, the thickening agent is present at about 1-10%, 1-5% or about 1-3% such as about 1, 2, 3, 4, 5, 6, 7, 8, 9 or 10% w/w and all numbers in between such as about 2.5% w/w.

The present viscous solution or gel formulation may optionally include at least one antioxidant and/or one chelating agent and/or preservative.

In addition, the topical formulations useful in the present invention can also comprise a pH adjusting agent. In one particular embodiment, the pH adjusting agent is a base. Suitable pH adjusting bases include bicarbonates, carbonates, and hydroxides such as alkali or alkaline earth metal hydroxide as well as transition metal hydroxides. Alternatively, the pH adjusting agent can also be an acid, an acid salt, or mixtures thereof. Further, the pH adjusting agent can also be a buffer. Suitable buffers include citrate/citric acid buffers, acetate/acetic acid buffers, phosphate/phosphoric acid buffers, formate/formic acid buffers, propionate/propionic acid buffers, lactate/lactic acid buffers, carbonate/carbonic acid buffers, ammonium/ammonia buffers, and the like. The pH adjusting agent is present in an amount sufficient to adjust the pH of the composition to between about pH 4.0 to about 10.0, more preferably about pH 6.9 to about 9.8. In certain embodiments, the unadjusted pH of the admixed components is between 8 and 10, such as 9, without the need for the addition of any pH adjusting agents.

In certain aspects, the pain reliever formulation or viscous solution comprising a non-steroidal anti-inflammatory drug (NSAID) has a viscosity of at least 100 cps. Preferably, the gel formulation has a viscosity of at least 500 cps. More preferably, the formulation has a viscosity of at least 1000 cps. In other embodiments, the viscosity is about 1000-10,000 cps, or about 10,000-15,000 cps, or about 15,000-20,000 cps. In a preferred embodiment, the viscous solution or formulations has a viscosity of about 800-5000 cps, such as about 800, 900, 1000, 2000, 3000, 4000, or 5000 cps and all numbers in between. For example, the formulation has a viscosity of about 900, 925, 950, 975, 1000, 1025, 1050, 1075, 1100, 1125, 1150, 1175, 1200, 1225, 1250, 1275, 1300, 1325, 1350, 1375, 1400, 1425, 1450, 1475, 1500, 1525, 1550, 1575, 1600, 1625, 1650, 1675, 1700, 1725, 1750, 1775, 1800, 1825, 1850, 1875, 1900, 1925, 1950, 1975, or 2000 cps. Alternatively, the formulation has a viscosity of about 0-25 cps or 3-25 cps.

A "viscous solution" includes a solution having a viscosity greater than that of water (*i.e.,* 1.0020 cps at 20°C) or as otherwise known in the art.

In one embodiment, the topical pain reliever formulation comprises, consists essentially of, or consists of:
(i) diclofenac sodium;
(ii) DMSO;
(ii) ethanol;
(iii) propylene glycol;
(v) a thickening agent;
(vi) optionally glycerol; and
(vii) water.

In another embodiment, the formulation is used for treating signs and symptoms of osteoarthritis in a subject suffering from articular pain, by topical administration to an afflicted joint area of a subject a therapeutically effective amount of the formulation.

In another embodiment, the topical formulation comprises, consists essentially of, or consists of:
(i) about 1-2% w/w diclofenac sodium;
(ii) about 40-50 % w/w DMSO;
(iii) about 23-29% w/w ethanol;
(iv) about 10-12% w/w propylene glycol;
(v) about 1-10% w/w thickener (such as hydroxypropyl cellulose); and
(vi) water to make 100% w/w.

In yet a further embodiment, the present invention provides a topical formulation comprising, consisting essentially of, or consisting of: a diclofenac sodium solution and at least one thickening agent, which can be selected from cellulose polymer, a carbomer polymer, a carbomer derivative, a cellulose derivative, polyvinyl alcohol, poloxamers, polysaccharides, and mixtures thereof.

In an aspect of the above embodiments, the thickening agents can be selected from cellulose polymers, carbomer polymers, a carbomer derivative, a cellulose derivative, polyvinyl alcohol, poloxamers, polysaccharides, and mixtures thereof.

In another aspect of the above embodiments, diclofenac sodium is present at about 2% w/w; DMSO is present at about 45.5% w/w; ethanol is present at about 23-29% w/w; propylene glycol is present at about 10-12% w/w; hydroxypropylcellulose (HY119) is present at about 0-6% w/w; glycerol is present at about 0-4%, and water is added to make 100% w/w. In other aspects, there is no glycerol in the gel formulation and hydroxypropylcellulose (HY119) is present at about 1-6% w/w. In further aspects, the end viscosity of the gel is about 500-5000 centipoise.

A feature of the above gel formulations is that when such formulations are applied to the skin, the drying rate is quicker and transdermal flux is higher than previously described compositions, such as those in U.S. Patent Nos. 4,575,515 and 4,652,557. Additional features of the preferred formulations include decreased degradation of diclofenac sodium, which degrades by less than 0.04% over the course of 6 months and a pH of 6.0-10.0, for example around pH 9.0. In one aspect, the topical formulation is described in U.S. Application No. 12/134,121, incorporated herein by reference.

In another embodiment, the topical formulation comprises, consists essentially of, or consists of:
(i) about 1-2% w/w diclofenac sodium;
(ii) about 40-50% w/w DMSO;
(iii) about 10-12 % w/w propylene glycol;
(iv) about 1-30 % w/w ethanol;
(v) optionally glycerine;
(vi) water; and
(vii) at least one thickening agent selected from the group consisting of a cellulose polymer, a carbomer polymer, a carbomer derivative, a cellulose derivative, hydroxypropyl cellulose and mixtures thereof.

Preferably the topical formulation has a viscosity of about 500 - 5000 cps, such as a viscosity of at least 1000 cps. In other embodiments, the viscosity is about 1000-10,000 cps, or about 10,000-15,000 cps, or about 15,000-20,000 cps. In a preferred embodiment, the viscous solution or formulations has a viscosity of about 800-5000 cps, such as about 800, 900, 1000, 2000, 3000, 4000, or 5000 cps and all numbers in between.

In one non-limiting example, the commercially available PENNSAID^{®} 1.5% topical solution is packaged into a container with a metering pump. Instead of counting 40 drops for one dose with the current commercial container, one single actuation delivers the claimed unit dose of 1.2 mL.

In another non-limiting example, a viscous formulation, with 2% diclofenac sodium and a thickening agent, is filled into containers with metering pumps. One actuation accurately delivers 1 gram of the viscous formulation, which is half the claimed dose.

### III. Methods of Use

The topical pain reliever formulations are particularly suited for use in treating pain-related diseases, disorders, or conditions, such as the treatment of the signs and symptoms of osteoarthritis (OA) chronically. It is also useful for the treatment of other chronic joint diseases characterized by joint pain, degeneration of articular cartilage, impaired movement, and stiffness. Suitable joints include the knee, elbow, hand, wrist and hip. Preferably, the joint includes a knee.

Due to the properties of higher flux and *in vivo* absorption, it is believed that the formulations of the present invention can be administered at lower dosing than previously described formulations. In particular, the compositions of the invention can be used at twice a day dosing or once a day dosing in the treatment of OA. This is a significant improvement as lower dosing is associated with better patient compliance, an important factor in treating chronic conditions.

Suitable amounts per administration will generally depend on the size of the joint, which varies per individual and per joint, however a suitable amount may range from 0.5 µl/cm² to 4.0 µl/cm². Preferably the amount ranges from 2.0 to 3.0 µl/cm². In a preferred aspect, the dose is 1, 2, 3, or 4 times daily with each dose being between 0.1-4 mL. In a preferred aspect, the dose is 2 times a day at 2 mL per dose (a total of 4 mL/daily.)

### IV. Pharmacokinetic Properties

*In vivo* tools can demonstrate whether changes in a topical viscous solution affect local delivery. The FDA has identified four potential technologies to investigate:
(1) Pharmacokinetic studies may be used to demonstrate efficacy and/or to determine product performance of topical viscous solutions. Bioequivalence studies with pharmacokinetic or clinical endpoints may be conducted to demonstrate equivalence between two different topical viscous solutions. For many topical viscous solutions, the amount of drug reaching the systemic circulation can be detected in the plasma and compared between topical viscous solutions, although its relationship to local delivery is still unknown.
(2) Skin Stripping removes the top layers of the skin for assay of drug concentration.
(3) Microdialysis involves inserting a small semipermeable capillary tube into the dermis about 1000 mm under the skin. The capillary tube is permeable to the drug; therefore, as perfusion fluid flows through the capillary, it takes up drug from the extracellular fluid of the tissue.
(4) Near Infrared Spectroscopy detects a unique signal that indicates the concentration of a particular drug and offers the possibility of a noninvasive assay of drug delivery to the skin.

*See* Critical Path Opportunities for Generic Drugs, Office of Generic Drugs, Office of Pharmaceutical Science, Center for Drug Evaluation and Research, dated May 1, 2007 available on the FDA.gov website. A skin irritation and sensitization study may also assist in determining bioequivalence between topical viscous solutions, especially if the differences involve potential penetration enhancers. *See* FDA Draft Guidance on Diclofenac Sodium, Revised June 2011.

The viscous solution, a topical diclofenac preparation, disclosed herein comprises a therapeutically effective amount of diclofenac sodium such that the patient quickly attains sufficient plasma concentrations of diclofenac (e.g., within about 4-12 hours such as about 4, 5, 6, 7, 8, 9, 10, 11 or 12 hours) after administration of the solution and maintains pain relief for about 12 hours after administration of the solution. Although systemic circulation of diclofenac detected in plasma does not directly correlate to local delivery, the FDA, in some instances, relies on pharmacokinetic data to determine bioequivalence between two topical diclofenac solutions. If the 90% confidence intervals for the ratios of the geometric means (test: reference) of the AUC and Cₘₐₓ fall between 80% and 125%, the test is bioequivalent to the reference and is presumed to provide a similar therapeutic effect as the reference. *See* FDA Draft Guidance on Diclofenac Sodium, Revised June 2011.

The topical diclofenac preparation disclosed herein, when topically administered to a subject, may produce a plasma profile characterized by a mean Cₘₐₓ (peak plasma concentration) for diclofenac sodium from about 10.0 ng/mL to about 25.0 ng/mL. In another embodiment, the mean Cₘₐₓ for diclofenac sodium may range from about 12.4 ng/mL to about 19.5 ng/mL. In an additional embodiment, the mean Cₘₐₓ for diclofenac sodium may be about 12.5, 13.0, 13.5, 14.0, 14.5, 15.0, 15.1, 15.2, 15.3, 15.4, 15.5, 15.6, 15.7, 15.8, 15.9, 16.0, 16.5, 17.0, 17.5, 18.0, 18.5, 19.0 or 19.5 ng/mL. Moreover, the mean Cₘₐₓ for diclofenac sodium at steady state may range from about 12.0 ng/mL to about 30.0 ng/mL, or from about 15.6 ng/mL/mg to about 25.0 ng/mL/mg. In an additional embodiment, the mean Cₘₐₓ for diclofenac sodium at steady state may be about 15.5, 16.0, 16.5, 17.0, 17.5, 18.0, 18.5, 19.0, 19.5, 19.6, 19.7, 19.8, 19.9, 20.0, 20.5, 21.0, 21.5, 22.0, 22.5, 23.0, 23.5, 24.0, 24.5 or 25.0 ng/mL.

In an additional embodiment, the topical diclofenac preparation, when topically administered to a subject, may produce a plasma profile characterized by a mean AUC₀₋₁₂ for diclofenac sodium from about 60.0 ng•hr/mL to about 140.0 ng•hr/mL. In a further embodiment, the mean AUC₀₋₁₂ for diclofenac sodium may be from about 76.0ng•hr/mL to about 124.0 ng•hr/mL. In another embodiment, the mean AUC₀₋₁₂ for diclofenac sodium may be about 75.0, 80.0, 85.0, 90.0, 91.0, 92.0, 93.0, 94.0, 94.5, 95.0, 95.5, 96.0, 96.5, 97.0, 97.5, 98.0, 99.0, 99.5, 100.0, 105.0, 110.0, 115.0, 120.0 or 125.0 ng•hr/mL.

In an additional embodiment, the topical diclofenac preparation, when topically administered to a subject, may produce a plasma profile characterized by a mean AUC₀₋₂₄ for diclofenac sodium from about 100 ng•hr/mL to about 300 ng•hr/mL. In a further embodiment, the mean AUC₀₋₂₄ for diclofenac sodium may be from about 150 ng•hr/mL/mg to about 250 ng•hr/mL. In another embodiment, the mean AUC₀₋₂₄ for diclofenac sodium may be about 150, 155, 160, 165, 170, 175, 180, 185, 190, 191, 192, 193, 194, 195, 196, 197, 198, 199, 200, 205, 210, 215, 220, 225, 240, 235, 240, 245 or 250 ng•hr/mL. Additionally, the mean AUC₀₋₂₄ for diclofenac sodium at steady state may range from about 200 ng•hr/mL to about 450 ng•hr/mL, or from about 250 ng•hr/mL to about 405 ng•hr/mL. In another embodiment, the mean AUC₀₋₂₄ for diclofenac sodium at steady state may be about 250, 260, 270, 280, 290, 300, 310, 311, 312, 313, 314, 315, 316, 317, 318, 319, 320, 321, 322, 323, 324, 325, 330, 340, 350, 360, 370, 380, 390, 400 or 405 ng•hr/mL.

In a further embodiment, the topical diclofenac preparation, when topically administered to a subject, may produce a plasma profile characterized by a median Tₘₐₓ (time to peak plasma concentration) for diclofenac sodium from about 4.0 hours to about 12.0 hours. In an alternate embodiment, the median Tₘₐₓ for diclofenac sodium may be from about 6.0 hours to about 10.0 hours. In another embodiment, the median Tₘₐₓ for diclofenac sodium may be about 4.0, 4.5, 5.0, 5.5, 6.0, 6.5, 7.0, 7.5, 8.0, 8.5, 9.0, 9.5, 10.0, 10.5, 11.0, 11.5 or 12.0 hours. Moreover, the median Tₘₐₓ for diclofenac sodium at steady state may range from about 0 hours to about 12.0 hours, or from about 0.5 hours to about 8.0 hours relative to the time of administration of the last dose. In another embodiment, the median Tₘₐₓ for diclofenac sodium at steady state may be about 0.5, 1.0, 1.5, 2.0, 2.5, 3.0, 3.5, 4.0, 4.5, 5.0, 5.5, 6.0, 6.5, 7.0, 7.5 or 8.0 hours.

In certain aspects, the methods and systems of the present invention provide upon application of the topical diclofenac preparation in a viscous solution to the knee of a patient an AUC₀₋₂₄ for diclofenac of about 195.51 ± 166.03 ng•h/mL.

In certain aspects, the methods and systems of the present invention provide upon application of the topical diclofenac preparation in a viscous solution to the knee of a patient a Cₘₐₓ for diclofenac of about 15.57 ± 12.96 ng/mL.

In certain aspects, the methods and systems of the present invention provide upon application of the topical diclofenac preparation in a viscous solution to the knee of a patient an AUC₀₋₂₄ at steady state for diclofenac of about 319.51 ± 162.36 ng•h/mL.

In certain aspects, the methods and systems of the present invention provide upon application of the topical diclofenac preparation in a viscous solution to the knee of a patient a Cₘₐₓ at steady state for diclofenac of about 19.79 ± 10.12 ng/mL.

In certain aspects, the methods and systems of the present invention provide upon application of the topical diclofenac preparation in a viscous solution to the knee of a patient a mean AUC₀₋₁₂ for diclofenac of about 76.0 ng•hr/mL to about 124.0 ng•hr/mL.

In certain aspects, the topical diclofenac preparation described herein achieves the plasma profiles disclosed herein when administered from hand pump 100 (FIG. 1). In one aspect, the hand pump delivers 0.5-5 mL per pump action, such as 1, 2, 3, 4, or 5 mL per pump action. In a preferred aspect, the pump action is 1 mL per pump.

In certain aspects, the present invention provides a method for treating the signs and symptoms of osteoarthritis of the knee of a human patient with a topical diclofenac preparation, the method comprising:
dispensing a therapeutically effective amount of diclofenac from the topical diclofenac preparation packaged in a pharmaceutically acceptable hand pump;
applying the therapeutically effective amount of diclofenac to the knee; and, wherein the patient attains therapeutic blood levels of diclofenac within 4 to 12 hours such as 4, 5, 6, 7, 8, 9, 10, 11 or 12 and all fractions in between after administration of the preparation and maintains pain relief for about 12 hours after administration of the preparation.

In one aspect, patients are given the following instructions. 1) Remove the pump cap. 2) To prime the pump, while holding the bottle in an upright but tilted position, press the pump down firmly and fully several times into a paper towel or tissue until some study drug comes out. 3) After priming the pump, the bottle is ready to use and does not need to be primed again. 4) To dispense the drug, press the pump head down firmly and fully to dispense the drug into the palm of the hand. Release the pump head. 5) Apply the drug evenly over the entire application area.

### V. Examples

**Example** 1 illustrates the performance evaluation of a 100 mL Rexam Sof'Bag bottle with 1 mL dispensing pump for diclofenac sodium topical 2.0% w/w.

### 1. Summary

The current study was performed to evaluate the dose delivery attributes of the Rexam Sof'Bag container with a metering pump for diclofenac sodium topical gel 2.0% w/w. The study was conducted on 16 containers over a one month (16 business days) period. Results show that the container, when filled to full capacity, is capable of consistently delivering the required number of doses with high precision. The mean unit dose weight delivered from each container varied from 0.97 to 0.99 g, which is well within 5% of the target dose (1.00 g). Unit dose weight standard deviations from each bottle were stable at 0.01 g.

### 2. Introduction

Diclofenac sodium topical gel 2.0% w/w is a drug under investigation for the treatment of the signs and symptoms of osteoarthritis of the knee(s).

According to FDA guidance document "Container Closure Systems for Packaging Human Drugs and Biologics," container performance includes "functionality and/or drug delivery" studies. This study was conducted to evaluate the following performance aspects: container priming parameters, delivery accuracy and precision, number of conforming doses, percentage of restitution, residual volume, and waste volume.

### 3. Materials, Equipment, Methods and Procedures

The study was conducted with a Rexam bottle and pump head. The inner pouch of the Rexam 100 mL bottle has a pressure rating of a half bar (7.2 psi). All instruments, including pressure gauge and balance were within calibration during this study.

Delivery attributes were studied over a total of sixteen (16) business days. The containers were filled, pumps were primed, and the first two dispensing sessions were performed. Containers were stored at room temperature during this period.

### 4. Results and Discussion

### 4.1. Container fill weight and deliverable

Bottles were manually inflated at 6 psi prior to filling. Each container was filled to full capacity: just below the bottom of bottle neck and top of the shoulder. The container weights before and after filling, and before and after dispensing were recorded. Container fill capacity and deliverable weights were calculated. The total collected sample weight from each container was also calculated by summation of individual unit dose weights. Key statistics are summarized below in Table 1.

| Table 1. Overall container fill capacity and deliverable | | | | | | | | | |
|---|---|---|---|---|---|---|---|---|---|
| | Empty container weight (g) | Initial gross weight (g) | Total fill weight (g) | End gross weight (g) | Residual¹ (g) | Total delivered (g) | Total collected (g) | Restitution (%)² | Yield (%) |
| Mean | 45.74 | 179.79 | 134.06 | 50.19 | 4.46 | 129.60 | 128.81 | 96.08 | 99.39 |
| STDEV | 0.04 | 1.20 | 1.21 | 0.75 | 0.74 | 1.58 | 1.68 | 0.59 | 0.29 |
| Minimum | 45.67 | 177.31 | 131.57 | 49.09 | 3.34 | 126.51 | 125.69 | 94.94 | 98.51 |
| Maximum | 45.84 | 181.75 | 136.08 | 51.62 | 5.88 | 132.34 | 131.51 | 96.93 | 100.09 |
| 1 Residual: Product left in bottle after completion of dispensing. | | | | | | | | | |
| 2 Restitution: Percentage of total collected in total fill weight. | | | | | | | | | |
| 3 Yield: Percentage of total collected in total delivered. | | | | | | | | | |

Under the filling conditions used in this study, the deliverable weight (total delivered) results meet the requirements of USP <698> for multiple-unit containers. The average deliverable weight is NLT 100% of 120 g minimum requirement, and no individual is less than 95% of minimum.

### 4.2. Priming requirement

The initial data collected at the beginning of bottle dispensing was used to assess the number of actuations required to fully prime the pump. Data indicated that once product is discharged, the next actuation always results in a full dose.

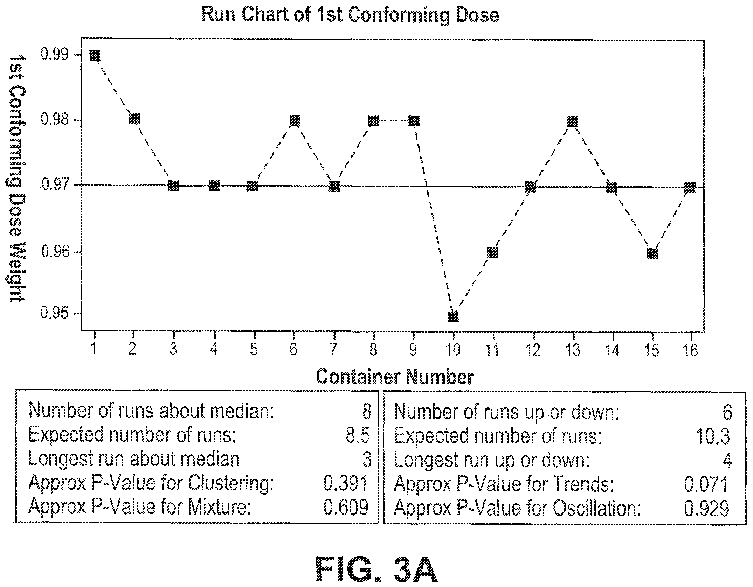

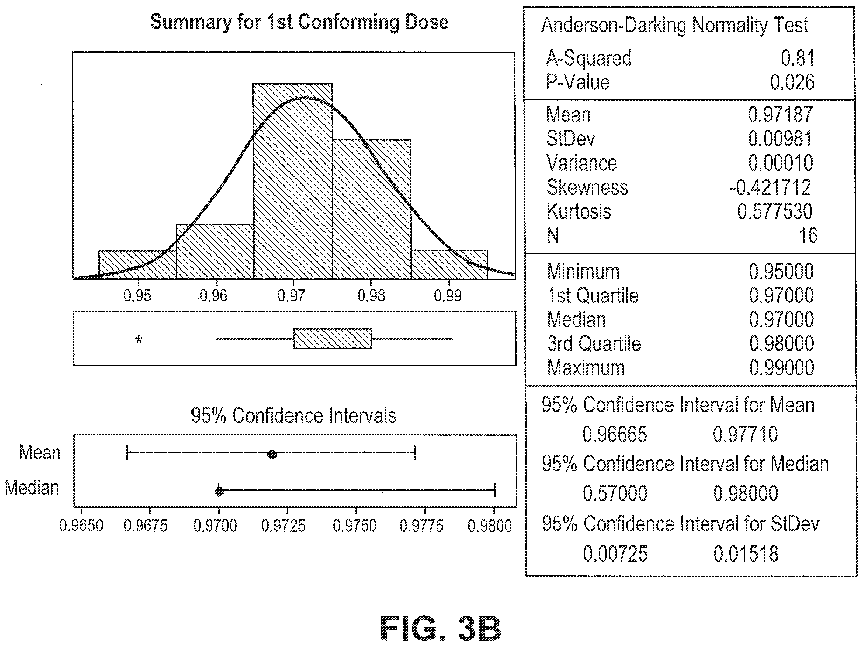

FIGS. 3A-3B show a run chart (FIG. 3A) and a statistical summary of the first conforming dose from all containers (FIG. 3B).

The number of priming actuations depends on container fill level. In this study, all containers were filled to full capacity, and two, three, four, or five actuations were required to fully prime the pump, depending on how full the bottles are filled.

Study results demonstrated that each bottle was capable of delivering more than 120 conforming doses. For consistency and comparison across all containers, only the first 120 consecutive doses after priming are analyzed in this session. Conforming doses during priming and at the end are described in 4.4.

### 4.3.1. Overall statistics

Overall statistics are listed in Table 2 below. Across all 16 containers, dose number 33 was consistently the lowest dose among the 120 unit doses. This was caused by solvent evaporation through the pump head after standing for two weeks.

| Table 2: Statistical summary of individual dose weight from all containers | |
|---|---|
| Statistics | Weight (g) |
| Individual Container - Maximum Mean | 0.99 |
| Individual Container - Minimum Mean | 0.97 |
| All Containers - Mean | 0.98 |
| All Containers - Standard Deviation | 0.01 |
| Maximum Single Actuation | 1.00 |
| Minimum Single Actuation (excluding dose No. 33, 1st dose after long gap period.)^{∗∗} | 0.93 |
| Minimum Single Actuation (dose No. 33) | 0.86 |
| ^{∗} Calculations were based on the first 120 consecutive doses after priming sessions. | |
| ^{∗∗} Unit dose No. 33 was dispensed on January 3rd, 2011, two weeks after the previous dose was dispensed. | |

### 4.3.2. Capability analysis

The current proposed product specification requires the average dose weight to be within 0.8-1.2 g. As shown in Table 2 above, mean unit dose weights are all well within specification. To further examine container pump performance, a capability analysis was performed on all unit doses sub-grouped by containers. Results demonstrated a high capability to consistently deliver unit doses in the 0.8-1.2 g range. Even when the range is set between 0.9-1.1 g, an overall Cpk of 2.89 and Ppk of 1.86 were obtained. The capability numbers will be higher if a special event, *i.e.* dose number 33, is excluded from the analysis.

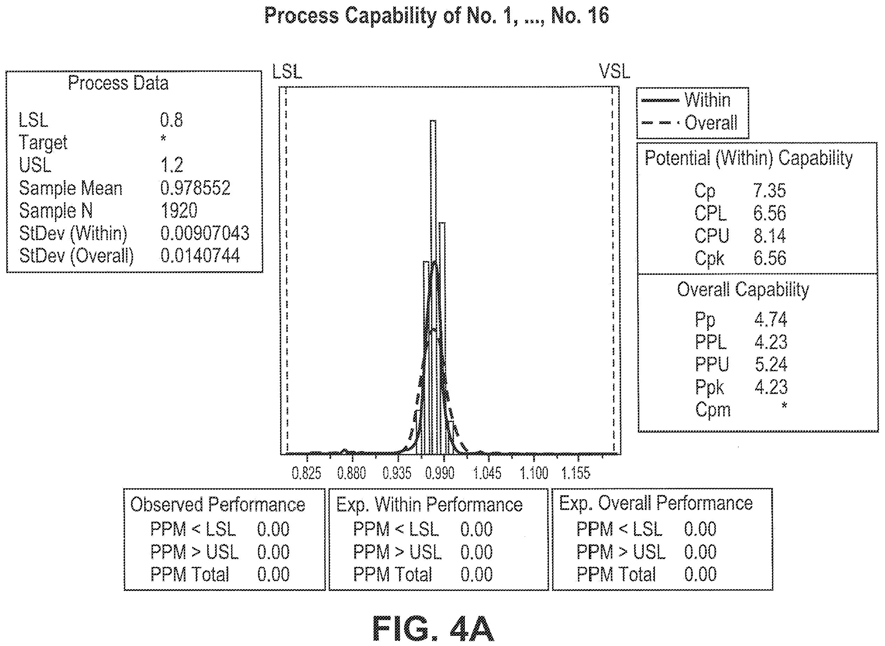

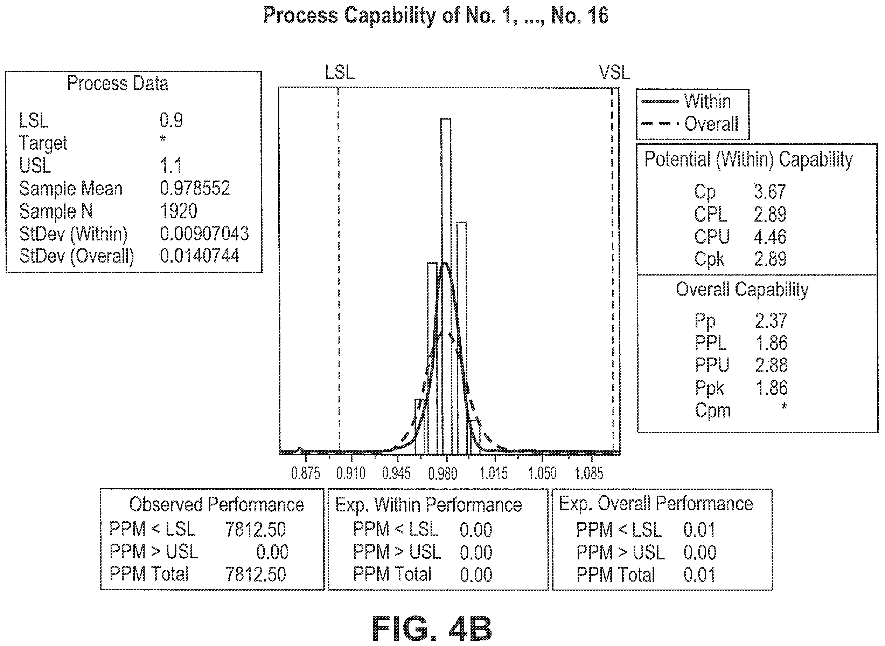

FIGS. 4A-4B summarize the capability analyses results.

Table 2A (below) shows comparative, empirical data among other pump designs that were tested. Three pumps were tested for priming requirements, accuracy and precision of dispensed amounts, residual product left in the bottles, etc. Accuracy and precision are reflected in the table.

| Table 2A: Comparison Data of Different Pump Designs | | | |
|---|---|---|---|
| All cells are in weight^{∗} (g) | Pump A (embodiment) | Pump B | Pump C |
| Individual Container - Maximum Mean | 0.99 | 1.04 | 1.23 |
| Individual Container - Minimum Mean | 0.97 | 1.02 | 1.12 |
| All Containers - Mean | 0.98 | 1.03 | 1.18 |
| All Containers - Standard Deviation | 0.01 | 0.03 | 0.06 |
| Maximum Single Actuation | 1.00 | 1.09 | 1.32 |
| Minimum Single Actuation | 0.93^{∗∗} | 0.77 | 0.51 |
| ^{∗} Calculations were based on the first 120 consecutive doses after priming sessions. | | | |
| ^{∗∗} Unit dose No. 33 was dispensed on January 3rd, 2011, two weeks after the previous dose was dispensed. | | | |

As shown in the table, the design of Pump A dispensed product more accurately *(i.e.,* within 0.02 g of 1.00 g) than Pump B *(i.e.,* within 0.03 g of 1.00 g) and Pump C *(i.e.,* within 0.18 g of 1.00 g). Accuracy in dispensing is important so that a product does not need to be re-formulated *(e.g.,* diluted, made stronger) in order for the pump to dispense the correct dose. Avoiding re-formulation saves time and effort in gaining regulatory acceptance of the product for market. An accurate and precise dispensed dose, in an inexpensive, reliable, and consumer-friendly dispenser, increases safety and effectiveness of the pain reliever.

Also as shown in the table, the design of Pump A dispensed product more precisely *(i.e.,* within a standard deviation of 0.01 g to its mean) than Pump B *(i.e.,* within a standard deviation of 0.03 g to its mean) and Pump C *(i.e.,* within 0.06 g to its mean). Precision in dispensing is important so that doses are repeatable. Similarly, the maximum and minimum single actuations of product should be close to the mean value so as to minimize dose outliers.

Pump A, which is in accordance with an embodiment of the invention, has thus been shown to be superior to Pumps B and C.

### 4.3.3. Process control analysis

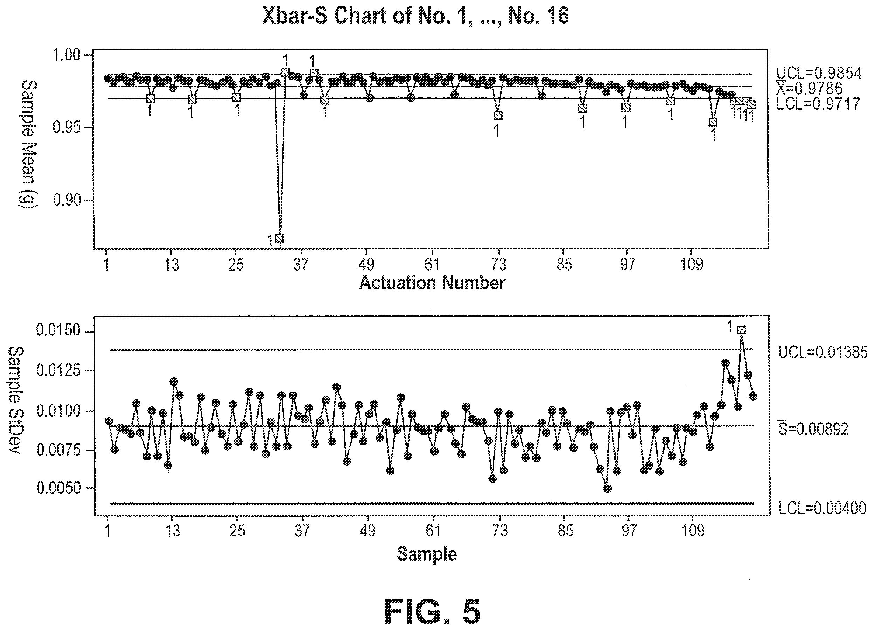

FIG. 5 is an X-bar/S chart that was used to evaluate the statistical control of the dispensing study. Each point in the upper chart represents the mean unit dose weight for all 16 containers, while the lower chart shows all corresponding standard deviations. The graph confirms the overall mean of 0.98 g per unit dose, with a standard deviation of 0.01g. Although several data points fall outside the ±3σ bands, the overall dispensing appears to be in good statistical control. Dose number 33 is a special cause event due to product evaporation after extended storage. Closer examination of the low doses before the late stage of dispensing showed that many of them coincided with the first unit dose in the morning, again attributed to slight evaporation. Average unit dose weight drop-off at the end is caused by low product quantity in the containers. Overall, unit dose weight remained stable and well within the 0.9-1.1 grange. FIG. 5 shows a statistical process control chart for unit dose weight

### 4.4. Number of conforming doses

All 120 doses analyzed above fall within product specification 0.8-1.2 g, they also meet the following tighter limits proposed in the protocol: within any 20 consecutive doses, no more than 2 doses out of the 0.9-1.1 g and none out of the 0.8-1.2 g range.

Based on above criteria, additional conforming doses were obtained. According to the protocol, three actuations were performed after the first product was discharged. As discussed in 4.2., all three actuations were conforming doses. More conforming doses were also obtained after the dispensing of the 120 unit doses. Overall, the total number of conforming doses ranged from 126 to 132.

### 4.5. Total waste

Total waste quantity includes the priming doses, nonconforming doses at the end, and residual left in the container. The average total waste was 6.58 g, with a standard deviation of 1.12 g.

### 4.6. Inflation pressure and till capacity

After inflation at 6 psi, all containers were filled to the same level. The average fill weight was 134.06 g, with a standard deviation of 1.21 g. The values varied from 131.57 - 136.08 g.

Separately from this protocol, a study was conducted to evaluate the impact of inflation pressure on fill capacity. An ANOVA analysis was performed and the results are summarized in below. A comparison of the mean capacity at each pressure was made against capacities at all other pressures, using Fisher's pairwise test. Statistical differences in fill capacities were seen between the following pressures: 3 and 5 & above, 4 and 5& above, 5 and 7 psi.

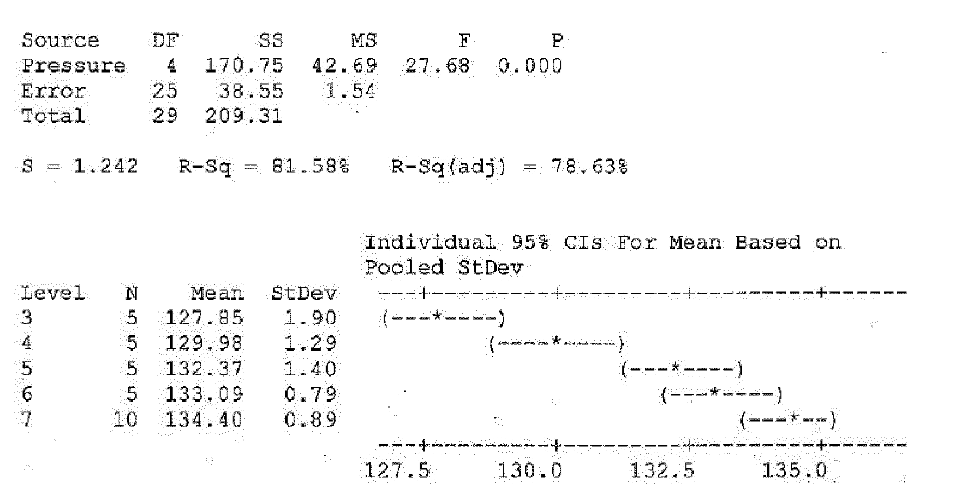

The foregoing is an ANOVA summary of the pressure impact on fill capacity.

### 4.7. Recommended minimum fill weight

The number of conforming doses is directly dependent on container fill weight and unit dose weight. Based on a conservative total waste quantity of 8 g, the following minimum fill weights are suggested at different unit dose weights.

| Table 3: Recommended minimum fill weights at different unit dose weights | | |
|---|---|---|
| Mean Unit dose weight (g) | Total conforming dose weight (g) | Minimum fill weight (g) |
| 0.95 | 114.0 | 122.0 |
| 0.98 | 117.6 | 125.6 |
| 1.00 | 120.0 | 128.0 |
| 1.02 | 122.4 | 130.4 |
| 1.05 | 126.0 | 134.0 |

### 5. Conclusion

The Rexam Sof'Bag 100 mL container with 1 mL dispensing pump is capable of delivering NLT 120 unit doses of Pennsaid Gel with high precision and accuracy. The mean unit dose weight delivered from all containers is 0.98 g, with a standard deviation of 0.01 g. Dose delivery attributes remained stable over 30 dispensing sessions & one month study period.

### 6. Deviations

The protocol specified a target fill weight of 132 g (range 131-133 g). However, in order to evaluate fill capacity variability at set pressure of 6 psi, all containers were filled to full capacity. The actual fill weights ranged from 131.57 - 136.08 g.

**Example 2:** Clinical Pharmacokinetic Analysis of a Topical Viscous Solution of 2.0 % w/w Diclofenac Sodium- Multiple Dose

A 2.0% w/w diclofenac sodium topical viscous solution of the present invention was applied to both knees (2 mL [40.4 mg] per knee), topically, every 12 ± 0.5 hours for 7.5 consecutive days in the fed condition. Subjects dispensed the solution from the hand Pump A of Example 1 and applied the topical viscous solution to clean dry skin. To avoid spillage, 2 mL (2 pumps) of the topical viscous solution was dispensed first into the hand and then onto the knee. The topical viscous solution was spread evenly around front, back and sides of knee. The procedure was repeated to the other knee allowing the application to dry completely.

The following pharmacokinetic parameters for diclofenac sodium were determined:
Day 1: The maximum observed plasma concentration (Cₘₐₓ), time to Cₘₐₓ (Tₘₐₓ) and
area under plasma concentration curve for the dosing interval 0 to 12 hours (AUC₀₋₁₂) and adjusted to time 0 to 24 hours (AUC₀₋₂₄);
Day 8: Steady-state parameters: AUC₀₋₂₄^{ss}_{;} maximum observed plasma drug concentration at steady-state (Cₘₐₓ ^{SS}), observed time to Cₘₐₓ at steady state (Tₘₐₓ ^{SS}).

The pharmacokinetic parameters for diclofenac were determined and calculated for the topical viscous solution over the dosing interval of 0 to 12 hours. The arithmetic mean and SD for the pharmacokinetic parameters for diclofenac calculated for the topical viscous solution are presented in the following Table 4:

**Table 4: Summary of Day 1 and Day 8 Diclofenac Pharmacokinetic Parameters for Subjects Completing the Study**

| **Parameters** | **2.0% w/w diclofenac sodium topical V.S. N=22 Mean (SD)** |
|---|---|
| **Day 1** | |
| **AUC₀₋₁₂ (ng•h/mL)** | 95.41 (80.16) |
| **AUC₀₋₂₄ (ng•h/mL)** | 190.82 (160.32) |
| **Cₘₐₓ (ng/mL)** | 15.63 (13.23) |
| **Tₘₐₓ (h) ^{a}** | 12 (6.00 - 12.00) |

| **Day 8** | |
|---|---|
| **AUC₀₋₂₄ (ng•h/mL)** | 315.47 (158.70) |
| **Cₘₐₓ ^{ss} (ng/mL)** | 19.53 (10.31) |
| **Tₘₐₓ ^{ss} (h)^{a, b}** | 1 (0.00 - 12.00) |

| | |
|---|---|
| ^{a}Tₘₐₓ and Tmax ^{SS} are presented as median (range) ^{b}Tₘₐₓ ^{SS} is relative to the time of administration of the last dose | |

After the initial dosing with the topical viscous solution, the mean diclofenac plasma concentrations increased rapidly within 6 hours and continued to rise until 24 hours. Steady-state measurements (before the morning dose on Days 2 through 8) showed that diclofenac concentrations remained elevated at approximately 20 ng/mL from 24 to 168 hours. Measurements immediately after final dosing at 168 hours showed diclofenac concentrations increased by approximately 10 fold with a gradual decline to 10 ng/mL at 192 hours (24 hours after the last dose).

**Example 3:** Clinical Pharmacokinetic Analysis of a Topical Viscous Solution of 2.0 % w/w Diclofenac Sodium- Multiple Dose

A 2.0% w/w diclofenac sodium topical viscous solution of the present invention was applied to both knees (2 mL [40.4 mg] per knee), topically, twice a day for 7.5 consecutive days. Total daily dose was approximately 162 mg. The topical viscous solution was supplied in metered-dose pump polypropylene bottles containing 120 mL each. Approximately 1 mL of the topical viscous solution was dispensed per pump. Subjects dispensed the solution from the hand Pump A of Example 1 and applied the topical viscous solution to clean dry skin. To avoid spillage, 2 mL (2 pumps) of the topical viscous solution was dispensed first into the hand and then onto the knee. The topical viscous solution was spread evenly around the front, back, and sides of knee. The procedure was repeated on the other knee, allowing the application to dry completely. Treatment was administered BID (6:00AM and 6:00PM). The following PK parameters for diclofenac were determined for both treatments:
Day 1: area under the plasma concentration curve for the first dosing interval 0 to 12 h (AUC₀₋₁₂) and adjusted to time 0 to 24 h (AUC₀₋₂₄), maximum observed plasma concentration (Cₘₐₓ), and time of observed maximum plasma concentration (Tₘₐₓ); Day 8: AUC for the last dosing interval adjusted to time 0 to 24 h (AUC₀₋₂₄ ^{SS}), Cₘₐₓ at steady state (Cₘₐₓ ^{SS}), Tmax at steady state (Tₘₐₓ ^{SS}).

The AUC₀₋₁₂ and AUC₀₋₁₂ ^{SS} were calculated for the topical viscous solution, over the dosing interval of 0 through 12 h on Day 1 and Day 8. So that a comparison between the exposure of each treatment could be assessed on a daily basis, AUC₀₋₂₄ and AUC₀₋₂₄ ^{SS} were calculated as AUC₀₋₁₂ and AUC₀₋₁₂ ^{SS} × 2 for the 2% w/w diclofenac sodium topical viscous solution.

After the initial dosing with the topical viscous solution, the mean diclofenac plasma concentration increased rapidly within 6 h and remained elevated until reaching a mean (SD) of 14.65 ng/mL (12.38) at 24 h. Steady-state measurements (before the morning dose on Days 2 through 8) showed that mean diclofenac concentrations remained between 12 and 16 ng/mL from 24 to 168 h. On Day 8, the topical viscous solution had an AUC₀₋₂₄ ^{SS} of 322.57 ng•h/mL (52% coefficient of variation [CV]) and a Cₘₐₓ ^{SS} of 19.99 ng/mL (51% CV). On Day 8, the topical viscous solution had an AUC₀₋₂₄ SS of 251.24 ng•h/mL (61% CV) and a Cₘₐₓ ^{SS} of 14.61 ng/mL (63% CV).

High inter-individual variability is characteristic of topical formulations. The arithmetic mean and SD for the PK parameters for diclofenac calculated for the topical viscous solution are presented in the following Table 5.

**Table 5: Summary of Day 1 and Day 8 Diclofenac PK Parameters for Subjects Completing the Trial**

| **Parameters** | **2.0% w/w diclofenac sodium topical V.S. N=29 Mean (SD)** |
|---|---|
| **Day 1** | |
| **AUC₀₋₁₂ (ng•h/mL)** | 99.54 (86.48) |
| **AUC₀₋₂₄ (ng•h/mL)** | 199.07 (172.96) |
| **Cₘₐₓ (ng/mL)** | 15.53 (12.98) |
| **Tₘₐₓ (h) ^{a}** | 12 (4.00 - 12.00) |

| **Day 8** | |
|---|---|
| **AUC₀₋₂₄ (ng•h/mL)** | 322.57 (167.81) |
| **Cₘₐₓ ^{ss} (ng/mL)** | 19.99 (10.15) |
| **Tₘₐₓ ^{ss} (h)^{a, b}** | 6.5 (0.00 - 12.00) |

| | |
|---|---|
| ^{a}Tₘₐₓ and Tₘₐₓ ^{SS} are presented as median (range) ^{b}Tₘₐₓ ^{SS} is relative to the time of administration of the last dose | |

### Example 4

Table 6 presents the diclofenac pharmacokinetic parameters of 51 healthy human volunteers topically administered a 2.0% w/w diclofenac sodium topical viscous solution of the present invention (dispensed from the hand Pump A of Example 1) to both knees (2 mL [40.4 mg] per knee), topically, twice a day for 7.5 consecutive days.

**Table 6**

| **Parameters** | **2.0% w/w diclofenac sodium topical V.S. N = 51** |
|---|---|
| **Day** 1 | |
| **AUC₀₋₂₄ (ng•h/mL)** | 195.51 ± 166.03 |
| **Cₘₐₓ (ng/mL)** | 15.57 ± 12.96 |

| **Day 8** | |
|---|---|
| **AUC₀₋₂₄ ^{ss} (ng•h/mL)** | 319.51 ± 162.36 |
| **Cₘₐₓ ^{ss} (ng/mL)** | 19.79 ± 10.12 |

| | |
|---|---|
| Data represents mean +/- Standard Deviation | |

It is understood that the examples and embodiments described herein are for illustrative purposes only and that various modifications or changes in light thereof will be suggested to persons skilled in the art and are to be included within the spirit and purview of this application and scope of the appended claims. All publications, patents, and patent applications cited herein are hereby incorporated by reference in their entirety for all purposes.

The application includes the following embodiments:
1. A method for administering a recommended dosage of a topical pain reliever in a viscous solution, the method comprising:
   actuating a hand pump, the actuating:
   a) causing a tappet having a rod connected thereto to compress a spring and the rod to slide with respect to a piston and enter a metering chamber holding a first predetermined amount of topical pain reliever in a viscous solution;
   b) causing an outlet valve of the metering chamber to open, which allows the first predetermined amount of topical pain reliever in the viscous solution to flow into a channel or opening of the rod; and then
   c) causing the piston to stroke when a limit stop of the tappet connects with a limit stop of the piston, wherein the first predetermined amount of topical pain reliever in the viscous solution is evacuated through an output duct of the tappet, which rises back up by action of the spring; and then
   d) causing the outlet value of the metering chamber to close and create a vacuum inside the metering chamber and to fill the chamber;
   e) optionally repeating steps a) through d) to deliver the recommended dose; and
   f) spreading the viscous solution on skin.
2. The method according to embodiment 1, wherein the topical pain reliever comprises sodium diclofenac.
3. The method according to embodiment 2, wherein the viscous solution is about 1.5% to about 3% by weight of sodium diclofenac
4. The method according to embodiment 3, wherein the viscous solution is about 2% by weight of sodium diclofenac.
5. The method according to embodiment 1, further comprising:
   aspirating the second predetermined amount of topical pain reliever from a sealed bag holding the viscous solution, thereby shrinking the bag.
6. The method according to embodiment 5, wherein the bag comprises metal and lined or coated with polyester or polyetheylene.
7. The method according to embodiment 6, wherein the metal includes aluminum.
8. The method according to embodiment 1, wherein hand pump is elongated and the actuating occurs when the elongated hand pump is held at an angle with respect to a gravitational vector.
9. The method according to embodiment 1, wherein hand pump is elongated and the actuating occurs when the elongated hand pump is upside-down with respect to a gravitational vector.
10. The method according to embodiment 1, wherein the skin covers a knee joint.
11. A method for treating signs and symptoms of osteoarthritis, the method comprising:
   depressing a hand pump to dispense 1/n of a dose of a topical pain reliever in a viscous solution from the hand pump, wherein n is a positive integer, wherein the hand pump is configured to dispense the 1/n of the dose within a tolerance specified by a corresponding label approved by a government regulatory agency; and
   spreading the topical solution on skin.
12. The method according to embodiment 11, wherein the hand pump includes a housing defining a metering chamber, a tappet having a rod connected thereto, which slides along a piston and enters the metering chamber causing an outlet valve of the metering chamber to open, which allows the first predetermined amount of topical pain reliever in a viscous solution to flow into a channel of the rod; and through an output duct of the tappet.
13. The method according to embodiment 11, wherein the dose tolerance is ±20 %.
14. The method according to embodiment 11, wherein the dose tolerance is ±10%.
15. The method according to embodiment 11, wherein n = 1, whereby the depressing of the hand pump dispenses an entire dose of the viscous topical pain reliever in a viscous solution.
16. The method according to embodiment 11, wherein n = 2, wherein the depressing of the hand pump twice dispenses a half-daily dose of the viscous topical pain reliever in a viscous solution.
17. The method according to embodiment 11, wherein the government regulatory agency is the United States Food and Drug Administration (FDA).
18. A dispensing system for a topical pain reliever in a viscous solution, the system comprising:
   a topical pain reliever in a viscous solution comprises sodium diclofenac;
   a sealed bag holding the viscous solution; and
   a hand pump in fluid communication with the viscous solution, the hand pump comprising:
      a pump housing defining a metering chamber;
   a tappet having a rod connected thereto to compress a spring and slide along a piston and enter a metering chamber holding a first predetermined amount of topical pain reliever in a viscous solution, wherein the metering chamber is sized for a throughput of 1/n dose of the sodium diclofenac, wherein n is a positive integer, wherein the first predetermined amount of topical pain reliever in the viscous solution is evacuated through an output duct of the tappet, which rises back up by action of the spring to dispense the 1/n of the dose within a tolerance specified by a corresponding label approved by a government regulatory agency.
19. The system according to embodiment 18, wherein the sealed bag holding the viscous solution excludes air, thereby allowing the hand pump and sealed bag to dispense the viscous solution from any orientation with respect to a gravity vector.
20. The system according to embodiment 18, wherein the viscous solution has a viscosity between about 500 and about 2000 centipoise.
21. The system according to embodiment 18, wherein the viscous solution has a viscosity between about 3 and about 25 centipoise.
22. The system according to embodiment 18, further comprising:
   a bottle enclosing the bag.
23. The system according to embodiment 18, wherein the viscous solution further comprises:
   dimethyl sulfoxide, an alkanol, propylene glycol, hydroxypropyl cellulose and water.
24. The system according to embodiment 18, wherein the viscous solution further comprises:
   dimethyl sulfoxide, an alkanol, propylene glycol, glycerin and water.
25. The system according to embodiment 18, wherein upon application of the topical pain reliever in a viscous solution to the knee of a patient, the topical pain reliever in a viscous solution provides an AUC₀₋₂₄ for diclofenac of about 195.51 ± 166.03 ng•h/mL.
26. The system according to embodiment 18, wherein upon application of the topical pain reliever in a viscous solution to the knee of a patient, the topical pain reliever in a viscous solution provides a Cₘₐₓ for diclofenac of about 15.57 ± 12.96 ng/mL.
27. The system according to embodiment 18, wherein upon application of the topical pain reliever in a viscous solution to the knee of a patient, the topical pain reliever in a viscous solution provides an AUC₀₋₂₄ at steady state for diclofenac of about 319.51 ± 162.36 ng•h/mL.
28. The system according to embodiment 18, wherein upon application of the topical pain .reliever in a viscous solution to the knee of a patient, the topical pain reliever in a viscous solution provides a Cₘₐₓ at steady state for diclofenac of about 19.79 ± 10.12 ng/mL.
29. The system of embodiment 18, wherein upon application of the topical pain reliever in a viscous solution to the knee of a patient, the topical pain reliever in a viscous solution provides a mean AUC₀₋₁₂ for diclofenac of about 76.0 ng•hr/mL to about 124.0 ng•hr/mL.
30. A method for treating the signs and symptoms of osteoarthritis of the knee of a human patient with a topical diclofenac preparation, the method comprising:
   dispensing a therapeutically effective amount of diclofenac from a topical diclofenac preparation packaged in a pharmaceutically acceptable hand pump;
   applying the therapeutically effective amount of diclofenac to the knee,
   wherein the patient attains therapeutic blood levels of diclofenac within about 4 to 12 hours after administration of the preparation and maintains pain relief for about 12 hours after administration of the preparation.
31. The method according to embodiment 30, wherein the therapeutically effective amount of diclofenac is about 1.5% to about 3% by weight of sodium diclofenac.
32. The method according to embodiment 31, wherein the therapeutically effective amount of diclofenac is about 1.5% by weight of sodium diclofenac.
33. The method according to embodiment 31, wherein the therapeutically effective amount of diclofenac is about 2.0% by weight of sodium diclofenac.
34. The method according to embodiment 30, wherein the therapeutically effective amount of diclofenac is dispensed by completely depressing the hand pump two times.
35. The method according to embodiment 34, wherein the dispensing and applying steps are performed two times a day.
36. The method according to embodiment 30, wherein upon application of the preparation to the knee, the preparation provides an AUC₀₋₂₄ for diclofenac of about 195.51 ± 166.03 ng•h/mL.
37. The method according to embodiment 30, wherein upon application of the preparation to the knee, the preparation provides a Cₘₐₓ for diclofenac of about 15.57 ± 12.96 ng•h/mL.
38. The method according to embodiment 30, wherein upon application of the preparation to the knee, the preparation provides an AUC₀₋₂₄ at steady state for diclofenac of about 319.51 ± 162.36 ng•h/mL.
39. The method according to embodiment 30, wherein upon application of the preparation to the knee, the preparation provides a Cₘₐₓ at steady state for diclofenac of about 19.79 ± 10.12 ng•h/mL.
40. The method according to embodiment 30, wherein upon application of the preparation to the knee, the preparation provides an AUC₀₋₁₂ for diclofenac of about 76.0 ng•hr/mL to about 124.0 ng•hr/mL.
41. The method according to embodiment 30, wherein the preparation further comprises: dimethyl sulfoxide, an alkanol, propylene glycol, glycerin and water.
42. The method according to embodiment 30, wherein the preparation further comprises: dimethyl sulfoxide, an alkanol, propylene glycol, hydroxypropyl cellulose and water.

## Claims

1. Use of a topical pain reliever in the treatment of the signs and symptoms of osteoarthritis, comprising:
depressing a hand pump to dispense a dose of the topical pain reliever in a viscous solution from the hand pump, wherein the hand pump is configured to dispense a metered dose within a tolerance specified by a corresponding label approved by a government regulatory agency;
spreading the viscous solution on a patient's knee;
wherein actuating the hand pump twice dispenses ½ of a daily dose of the topical pain reliever in a viscous solution,
wherein the daily dose is 4 actuations of the hand pump,
wherein one actuation of the hand pump delivers 1 gram of the viscous solution, and
wherein the hand pump is configured to contain at least 120 grams of the viscous solution.

2. The use of claim **1,** wherein the number of actuations is directly dependent on the container fill weight and the dose tolerance is 10%.

3. The use of claim **2,** wherein each actuation of the hand pump delivers a conforming amount of the viscous solution.

4. The use of any one of claims **1-3,** wherein the hand pump comprises a sealed bag holding the viscous solution that excludes air, thereby allowing the hand pump to dispense the viscous solution from any orientation with respect to a gravity vector.

5. The use of any one of claims **1-4,** wherein the hand pump is a water-tight hand pump.

6. The use of any one of claims **1-5,** wherein said use comprises dispensing the viscous solution from the hand pump and spreading the solution onto the knee of the patient wherein the patient is instructed to avoid exposure of the treated knee to natural or artificial sunlight while using said topical pain reliever.

7. The use of any one of claims **1-6,** wherein said use comprises dispensing the viscous solution from the hand pump and spreading the solution onto the knee of the patient wherein the patient is instructed to avoid taking unprescribed acetaminophen.

8. The use of any one of claims **1-7,** wherein said use comprises dispensing the viscous solution from the hand pump and spreading the solution onto the knee of the patient wherein the patient is instructed to avoid massaging the viscous solution into the knee and to avoid skin-to-skin contact between the knee and another person until the knee is dry.

9. The use of any one of claims **1-8,** wherein the viscous solution is an NSAID.

10. The use of any one of claims **1-9,** wherein the viscous solution comprises:
(i) about 1 -2% w/w diclofenac sodium;
(ii) about 40-50 % w/w DMSO;
(iii) about 23-29% w/w ethanol;
(iv) about 10-12% w/w propylene glycol;
(v) about 1-10% w/w thickener (such as hydroxypropyl cellulose); and
(vi) water to make 100% w/w.

11. The use of claim **10,** wherein upon application of the viscous solution to the knee, the viscous solution provides an AUC₀₋₂₄ for diclofenac of about 195.51 ± 166.03 ng•h/mL.

12. The use of claim **10,** wherein upon application of the viscous solution to the knee, the viscous solution provides a Cₘₐₓ for diclofenac of about 15.57 ± 12.96 ng/mL.

13. The use of claim **10,** wherein upon application of the viscous solution to the knee, the viscous solution provides an AUC₀₋₂₄ at steady state for diclofenac of about 319.51 ± 162.36 ng•h/mL.

14. The use of claim **10,** wherein upon application of the viscous solution to the knee, the viscous solution provides a Cₘₐₓ at steady state for diclofenac of about 19.79 ± 10.12 ng•h/mL.

15. The use of claim **10,** wherein upon application of the viscous solution to the knee, the viscous solution provides an AUC₀₋₂₄ for diclofenac of about 76.0 ng•hr/mL to about 124.0 ng•hr/mL.
